# EUROPEAN PATENT SPECIFICATION

(11) **EP 1 963 268 B1**
(45) Date of publication and mention of the grant of the patent: **01.12.2010**
(21) Application number: 06829342.2
(22) Date of filing: 06.12.2006
(51) Int. Cl.: C07D 211/18

(54) **DISUBSTITUTED PHENYLPIPERIDINES AS MODULATORS OF CORTICAL CATECHOLAMINERGIC NEUROTRANSMISSION**
DISUBSTITUIERTE PHENYLPIPERIDINE ALS MODULATOREN DER CORTICALEN CATECHOLAMINERGEN NEUROTRANSMISSION
PHENYLPIPERIDINES DISUBSTITUEES UTILES EN TANT QUE MODULATEURS DE LA NEUROTRANSMISSION CATECHOLAMINERGIQUE CORTICALE

(30) Priority: 07.12.2005 US 748077 P; 07.12.2005 SE 0502709
(43) Date of publication of application: 03.09.2008
(73) Proprietor: NSAB, Filial af NeuroSearch Sweden AB, Sverige, 2750 Ballerup (DK)
(72) Inventor: SONESSON, Clas, S-427 37 Billdal (SE); SWANSON, Lars, S-433 51 Öjersjö (SE); PETTERSSON, Fredrik, S-414 58 Göteborg (SE); WATERS, Nicholas, S-413 19 Göteborg (SE); WATERS, Susanna, S-413 19 Göteborg (SE)
(74) Representative: Abildgren, Michael Padkjaer
(86) International application number: PCT/EP2006/011712
(87) International publication number: WO 2007/065655

(56) References cited:
- EP-A- 1 419 773
- FR-A- 1 459 013
- US-A1- 2007 032 469

## Description

### Field of the invention

The present invention relates to the use of compounds which increase extracellular levels of catecholamines, dopamine and norepinephrine, in cerebral cortical areas of the mammalian brain, and more specifically to the use of 4-(ortho, para disubstituted phenyl)-1-piperidines, 4-(meta, para disubstituted phenyl)-1-piperidines, and 4-(meta, meta disubstituted phenyl)-1-piperidines for the treatment of central nervous system disorders.

### Background of the invention

The cerebral cortex encompasses several major regions that are involved in higher functions such as thought, feelings, memory and planning (Principles of Neural science, 2nd Edition, Elsevier Science Publishing co., Inc. 1985, pp 671 - 687). Biogenic amines, i.e. dopamine, norepinephrine and serotonin, are important for mammalian cortical function. The ascending dopamine and norepinephrine pathways innervate the cortex. The serotonergic neurons of the CNS, project to virtually all regions of the brain including the cerebral cortex (Fundamental Neuroscience, Academic press 1999, pp 207-212). Primary or secondary dysfunctions in the activity of these pathways lead to dysregulation of the activity at dopamine and norepinephrine and serotonin receptors in these brain areas and subsequently to manifestations of psychiatric and neurological symptoms.

The biogenic amines of the cortex modulate several aspects of cortical functions controlling affect, anxiety, motivation, cognition, attention, arousal and wakefulness (Neuropsychopharmacology, 5^{th} generation of Progress, Lippincott, Williams and Wilkins 2002, Chapter 34). Thus, the catecholamines dopamine and norepinephrine exert strong influence on the prefrontal cortical areas, the integrity of which is essential for the so-called executive cognitive functions, related to e.g. attention, planning of actions and impulse control (the role of the catecholamines in these respects is reviewed in Arnsten and Li, 2005, Biol Psychiatry; 57; 1377-1384). Norepinephrine is a major part in the circuitry regulating anxiety and fear and is thus believed to be dysregulated in anxiety disorders such as panic disorders, generalized anxiety disorder (GAD) and specific phobias (Sullivan et al 1999, Biol Psychiatry;46:1205-121). Concerning mood and affective functions, the usefulness of compounds facilitating particularly norepinephrine and serotonin neurotransmission in the treatment of depression and anxiety has strongly contributed to the widely-accepted concept that these neurotransmitters are both involved in the regulation of affective functions (Goodman & Gilman's The Pharmacological Basis of Therapeutics, Tenth Edition, McGraw-Hill, 2001).

In general, compounds specifically affecting the transmission of biogenic amines, more precisely monoamines, norepinephrine, dopamine and serotonin are successfully used to alleviate the affective, cognitive, or attentional symptoms in patients suffering from e.g. depression, anxiety and attention deficit hyperactivity disorders (ADHD).

Furthermore, the monoamine systems in the cortex are known to be directly or indirectly involved in the core symptoms of schizophrenia. Based on a synthesis of biochemical and genetic findings along with neuropsychological observations indicating dysfunction of specific cortical areas in schizophrenia, it has been proposed that this disorder emerges as various pathological etiologies converge upon cortical function leading to dysregulation of the cortical micro-circuitry, which is clinically manifested as the symptoms of schizophrenia (Harrison and Weinberger, 2005, Molecular Psychiatry; 10:40 - 68). This cortical micro-circuitry is regulated by several neurotransmitters, including glutamate, GABA, and dopamine.

### Description of Prior Art

Compounds belonging to the class of substituted 4-phenyl-piperidines have been reported previously. Among these compounds, some are inactive in the CNS, some display serotonergic or mixed serotonergic/dopaminergic pharmacological profiles while some are full or partial dopamine receptor agonists or antagonists with high affinity for dopamine receptors.

Fuller R. W. et al, J. Pharmacol. Exp. Therapeut. 218, 636, (1981) disclose substituted piperazines (*e.g*. 1-(m-trifluoro-methylphenyl)piperazine), which reportedly act as serotonin agonists and inhibit serotonin re-uptake. The comparative effects on the 5-hydroxyindole acetic acid concentrations in rat brain by 1-(p-chlorophenol)-piperazine are disclosed by Fuller R. W. et al, *Res. Commun. Chem. Patho*/*. Pharmacol.* 29, 201, **(1980).** Fuller R. Wet al, *Res. Commun. Chem. Pathol. Pharmacol.* 17, 551, **(1977)** disclose the comparative effects on the 3,4-dihydroxy-phenylacetic acid concentrations in rat brain by 1-(p-chlorophenol)-piperazine.

Stanislav, R. et al J. Heterocyclic Chem. 36, 1017, (1999) disclose "synthesis of piperidine analogs of 1-(3-chlorophenyl)piperazine, a well known serotonin ligand".

WO98/51668 disclose piperidine derivatives as neurotransmitter re-uptake inhibitors where the piperidine ring is substituted with oxime-ether groups

In addition, a number of substituted 4-phenyl-piperidines have been found in the literature, primarily as synthesis intermediates. The following compounds are found in Chemical Abstracts:

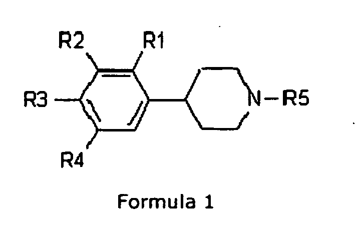

| Synthesis | | | | | | |
|---|---|---|---|---|---|---|
| R1 | R2 | R3 | R4 | R5 | Intermediate | Document |
| F | H | F | H | H | Yes | WO0051608 |
| | | | | | | US20050277647 |
| H | F | F | H | H | Yes | WO2004058727 |
| | | | | | | WO0071517 |
| | | | | | | WO0051608 |
| | | | | | | JP2000086603 |
| H | F | H | F | H | Yes | W00230936 |
| Cl | H | Cl | H | H | Yes | GB2083476A |
| | | | | | | US20060094707 |
| | | | | | | WO2004113298 |
| Cl | H | F | H | H | Yes | WO04113298 |
| H | F | Cl | H | H | Yes | WO0071517 |
| | | | | | | JP2000086603 |
| H | Cl | Cl | H | H | Yes | WO02051833 |
| | | | | | | WO0214271 |
| | | | | | | WO0071517 |
| | | | | | | JP2000086603 |
| | | | | | | EP0580398 |
| | | | | | | W09113865 |
| | | | | | | JP60146872 |
| H | CF3 | Cl | H | H | Yes | WO0146146 |
| CN | H | F | H | H | Yes | WO00027827 |
| | | | | | | WO0027817 |
| | | | | | | WO0025782 |
| | | | | | | W00006565 |
| | | | | | | J. Med. Chem. |
| | | | | | | (2000), 43(14), |
| | | | | | | 2703-2718 |
| CN | H | Cl | H | H | Yes | Tetrahedron, 2004, p.11367 |

US4415736 discloses 4-(2,3-dimethoxy-phenyl)-1-methyl-4-piperidinol as a synthesis intermediate.

It is known that compounds with formulae 2 (WO01/46145) and 3 (WO01/46146) possess dopaminergic stabilizer properties.

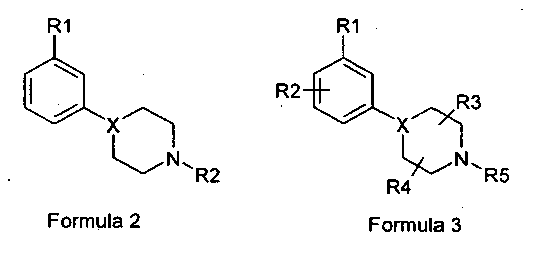

The prior art teaches that phenyl piperidines of WO01/46146 and WO01/46145 have a specific, efficacious, and characteristic effect on the metabolism of dopamine, measured as increases in tissue content of DOPAC (3,4-dihydroxyphenylacetic acid) in the striatum (see Table 1). This effect on subcortical dopamine metabolism is not the objective of the present invention.

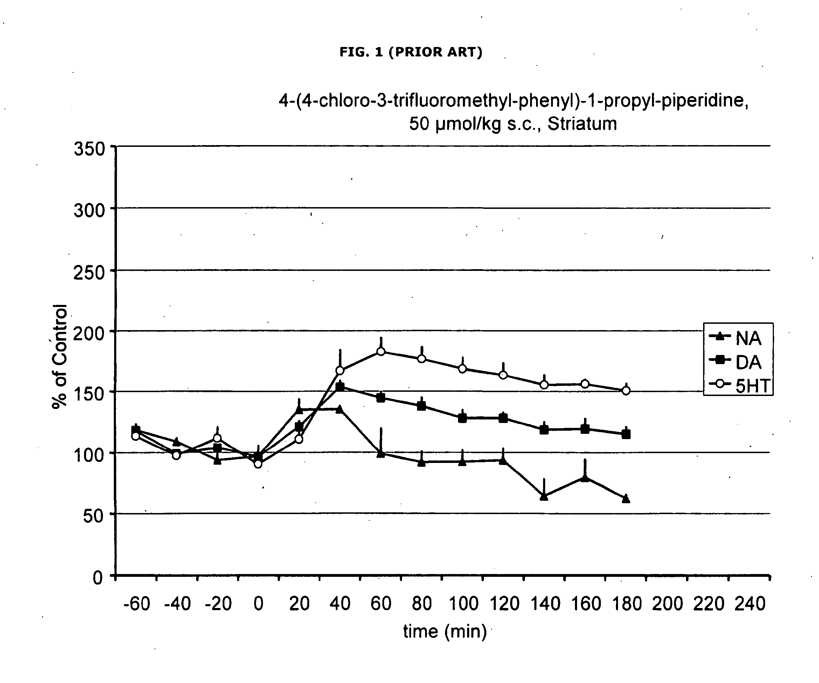

In addition, using a microdialysis technique it is shown that compounds from WO01/461 were found to increase extracellular levels of monoamines, (dopamine, norepinephrine and serotonin), with equal effects in both striatum and in cerebral cortical areas of the mammalian brain (See Figures 1 -8). In other words, the regionally selective properties of the compounds of the present invention between striatum and in cerebral cortical areas are not present in the prior art.

Thus, there is no guidance in either WO01/46146 or WO01/46145 on how to obtain compounds that increase norepinephrine and dopamine neurotransmission with a preference for the frontal cortex.

### Summary of the invention

The present invention concerns the unexpected discovery of the pharmacological effects of compounds of formula 4-6 on monoamines in the cerebral cortex, and the use of compounds of formula 4-6 as treatment for certain CNS disorders. By pharmacological testing *in vivo* in the rat it is demonstrated that the compounds of the present invention produce regionally selective increases in catecholamine levels in the frontal cortex. Due to the specific modulatory effects of the catecholamines on cortical functions related to cognition, attention and affect, the compounds of the invention can be used in the treatment of disorders characterised by dysfunctions in these areas. Thus, the compounds can be used in the treatment of cognitive disorders, ADHD, depression, and anxiety. The compounds can also be used to treat schizophrenia, which is characterised by dysfunctions of the cerebral cortex manifested in cognitive failure and psychosis.

### Detailed Description of the Invention

The following abbreviations will be used in the present invention:
NA: norepinephrine, NM: normetanephrine; DA: dopamine, DOPAC: 3,4-dihydroxyphenylacetic acid; 3-MT: 3-methoxytyramine; 5-HT: serotonin (5-hydroxytryptamine).

The present invention relates to new 4-(ortho, para disubstituted phenyl)-1-piperidines, 4-(meta, para disubstituted phenyl)-1-piperidines and 4-(meta, meta disubstituted phenyl)-1-piperidines in the form of free bases or pharmaceutically acceptable salts thereof, pharmaceutical compositions containing said compounds and use of said compounds in therapy, said groups of piperidines being claimed individually.

Specifically, the invention relates to a compound of Formula 4: 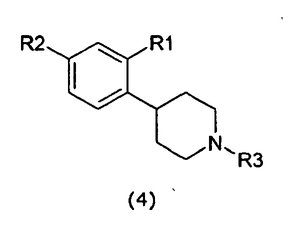 wherein:
R₁ is selected from the group consisting of -CN, -CF₃, CHF₂, F and Cl;
R₂ is selected from the group consisting of F and Cl;
R₃ is selected from the group consisting of H,
and the pharmaceutically acceptable salts thereof;
or a compound of formula 5: 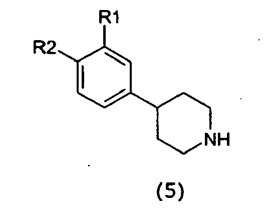 wherein:
R₁ is selected from the group consisting of -CN, -CF₃, CHF₂, F and Cl;
R₂ is selected from the group consisting of F and Cl;
and the pharmaceutically acceptable salts thereof;
or a compound of formula 6: 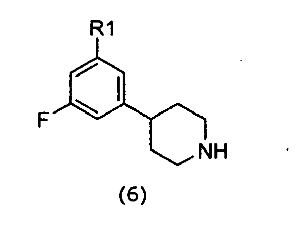 wherein:
R₁ is selected from the group consisting of F and Cl;
and the pharmaceutically acceptable salts thereof.

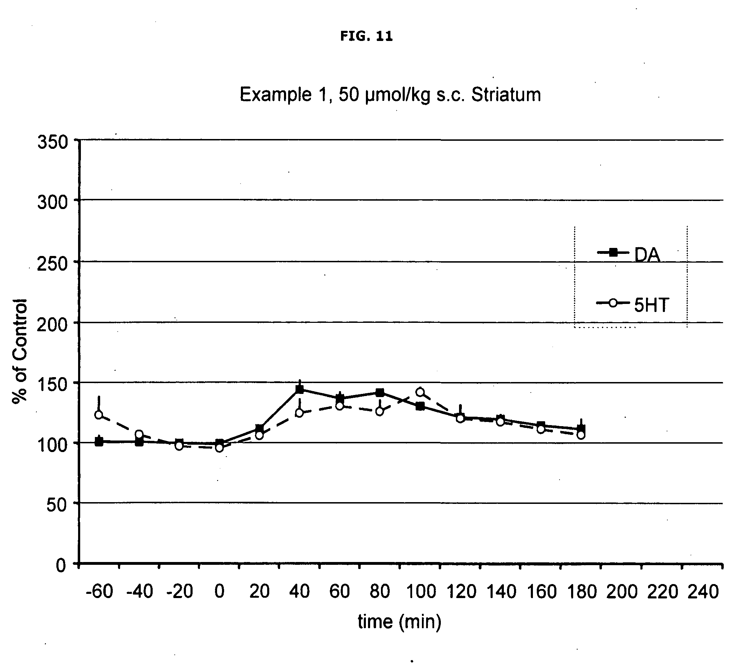

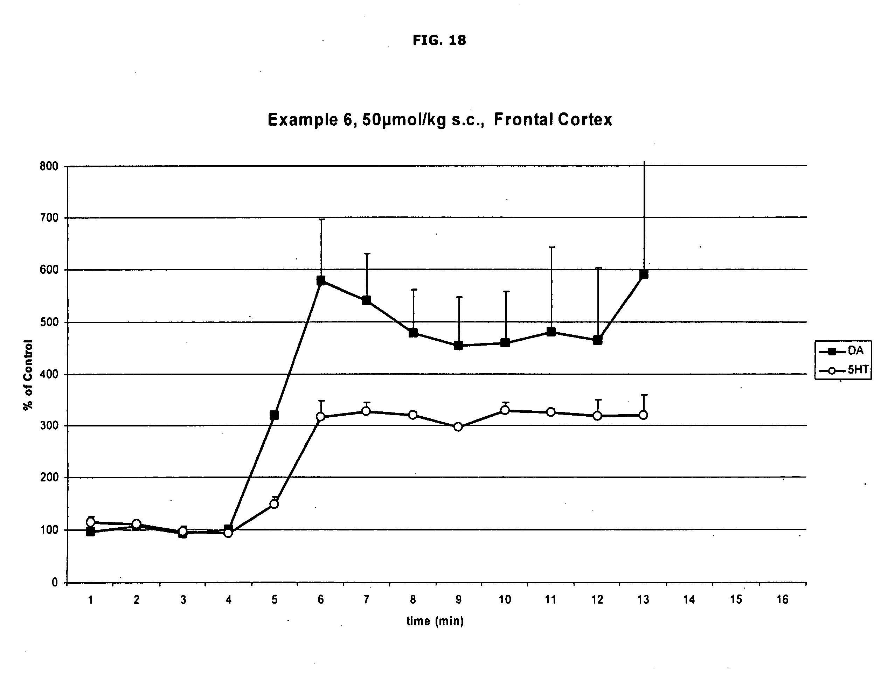

The compounds of formulae 4-6 have been found to increase the extracellular levels of norepinephrine and dopamine preferentially in the frontal cortex with no or substantially smaller effects in the striatum, as measured by the microdialysis technique. The unprecedented increase in cortical norepinephrine and dopamine of these compounds is illustrated in **FIGURE 11** - **22****.**

One aim of the present invention is to provide new compounds for therapeutic use, and more precisely compounds with modulation of dopamine and norepinephrine neurotransmission in the mammalian brain, including the human brain.

Another aim of the invention is to provide compounds with therapeutic effects after oral administration.

In a first embodiment, the present invention relates to compounds of formula 4-6 and pharmaceutically acceptable salts thereof, wherein R₁, R₂, R₃, are as defined above, with the provisos that;
in Formula (4) above,
R₁ and R₂ are not both F when R₃ is H;
R₁ and R₂ are not both Cl when R₃ is H;
R₁ is not Cl when R₂ is F and R₃ is H
R₁ is not -CN when R₂ is F and R₃ is H;
R₁ is not -CN when R₂ is Cl and R₃ is H;
in Formula (5) above,
R₁ and R₂ are not both F;
R₁ and R₂ are not both Cl;
R₁ is not F when R₂ is Cl;
R₁ is not -CF₃ when R₂ is Cl;
R₁ is not -CF₃ when R₂ is F;
and in Formula (6) above,
R₁ is not F;

Within this group of compounds, R₁ is preferably selected from the group consisting of F and Cl. More preferably, R₁ is selected from the group consisting of F.

In one embodiment, R₂ is selected from the group consisting of F and Cl. In another embodiment, R₃ is selected from the group consisting of H. Especially preferred compounds of the invention are those in which R₂ is F and R₃ is selected from the group consisting of H.

A preferred embodiment of the present invention is the secondary amines, i.e. wherein R₃ is H.

The preferred structures are:
4-(3-CHLORO-5-FLUOROPHENYL)PIPERIDINE
4-(3-CHLORO-4-FLUOROPHENYL)PIPERIDINE
4-(4-CHLORO-2-FLUOROPHENYL)PIPERIDINE
2-FLUORO-5-PIPERIDIN-4-YL-BENZONITRILE
2-CHLORO-5-PIPERIDIN-4-YL-BENZONITRILE
4-[3-(DIFLUOROMETHYL)-4-FLUOROPHENYL]PIPERIDINE
4-[4-CHLORO-3-(DIFLUOROMETHYL)PHENYL]PIPERIDINE
4-[2-(DIFLUOROMETHYL)-4-FLUOROPHENYL]PIPERIDINE
4-[4-CHLORO-2-(DIFLUOROMETHYL)PHENYL]PIPERIDINE
4-[4-FLUORO-2-(TRIFLUOROMETHYL)PHENYL]-PIPERIDINE
4-[4-CHLORO-2-(TRIFLUOROMETHYL)PHENYL]-PIPERIDINE

The invention also relates to any compound of any of Formulas 4 to 6 for use as a medicament.

The invention relates to the use of a compound of Formula 4-6 for the manufacture of a pharmaceutical composition for treatment of a disorder of the central nervous system. The preferred compounds for such a use (defined by R₁, R₂ and R₃) are those given above. The compounds listed in the provisos above are preferably excluded. Preferred compounds for use for the manufacture of a pharmaceutical composition for treatment of a disorder of the central nervous system are:
4-(3-CHLORO-5-FLUOROPHENYL)PIPERIDINE
4-(3-CHLORO-4-FLUOROPHENYL)PIPERIDINE
4-(4-CHLORO-2-FLUOROPHENYL)PIPERIDINE
2-FLUORO-5-PIPERIDIN-4-YL-BENZONITRILE
2-CHLORO-5-PIPERIDIN-4-YL-BENZONITRILE
4-[3-(DIFLUOROMETHYL)-4-FLUOROPHENYL]PIPERIDINE
4-[4-CHLORO-3-(DIFLUOROMETHYL)PHENYL]PIPERIDINE
4-[2-(DIFLUOROMETHYL)-4-FLUOROPHENYL]PIPERIDINE
4-[4-CHLORO-2-(DIFLUOROMETHYL)PHENYL]PIPERIDINE
4-[4-FLUORO-2-(TRIFLUOROMETHYL)PHENYL]-PIPERIDINE
4-[4-CHLORO-2-(TRIFLUOROMETHYL)PHENYL]-PIPERIDINE
4-[4-FLUORO-3-(TRIFLUOROMETHYL)PHENYL]-PIPERIDINE
4-(3,5-DIFLUOROPHENYL)PIPERIDINE
4-(3,4-DIFLUOROPHENYL)PIPERIDINE
4-(3,4-DICHLOROPHENYL)PIPERIDINE
4-(4-CHLORO-3-FLUOROPHENYL)PIPERIDINE
4-(2,4-DIFLUOROPHENYL)PIPERIDINE
4-(2,4-DICHLOROPHENYL)PIPERIDINE
4-(2-CHLORO-4-FLUOROPHENYL)PIPERIDINE
5-FLUORO-2-PIPERIDIN-4-YL-BENZONITRILE
5-CHLORO-2-PIPERIDIN-4-YL-BENZONITRILE
4-[4-CHLORO-3-(TRIFLUOROMETHYL)PHENYL]-PIPERIDINE

The present invention further relates to a method for treating disorders of the central nervous system, by administrating a therapeutically active amount of a compound according to Formula 4-6 to a mammal, including a human being, suffering from such a disorder. The present invention also relates to a method for treating any disorder named herein, by administrating a therapeutically active amount of a compound according to Formula 4-6 to a mammal, including a human being, suffering from such a disorder.

The compounds according to the present invention possess norepinephrine, dopamine and to some extent serotonin-modulating properties and both they and their pharmaceutical compositions are useful in treating numerous central nervous system disorders including psychiatric disorders. Particularly, the compounds and their pharmaceutical compositions are used in the treatment of CNS disorders, particularly those in which the cortical monoaminergic systems are dysfunctional due to direct or indirect causes.

The compounds and compositions according to the invention can be used to treat cognitive disorders including neurodegenerative (e.g. dementia and age-related cognitive impairment) and developmental disorders, such as Autism spectrum disorders, ADHD, Cerebral Palsy, Gilles de la Tourette's syndrome, as well as cognitive disorders occurring as part of the core symptoms of schizophrenia.

The compounds and compositions according to the invention can be used to treat affective disorders including depression and bipolar disorder. They can also be used to treat schizophrenia and schizophreniform disorders.

The compounds and compositions according to the invention can be used to treat anxiety disorders including generalized anxiety disorder (GAD), specific phobias and panic disorder (PD)

They are further useful for treatment of sleep disorders.

The compounds according to the present invention have been shown to increase the extracellular levels of dopamine and norepinephrine in the cerebral cortex and in some cases also serotonin.

However, the compounds of the present invention do not have the specific, efficacious and characteristic effects on the metabolism of dopamine in the striatum that is the essential characteristic for the pharmacological actions of the compounds described in neither WO01/46146 nor WO01/46145. Thus the compounds of the present invention have a surprising and distinct pharmacology (see Table 1).

**Table 1: The increase in DOPAC levels (3,4-dihydroxyphenylacetic acid) in the rat striatum after systemic adminstration of test compound (100 µmol/kg s.c.). Expressed as the %-increase from control value. For method see the enclosed description.**

| | **DOPAC** |
|---|---|
| **Comparative Examples** | **%-increase** |
| 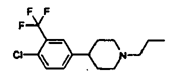 | + 94 |
| Example 9 of WO01/461 | |
| 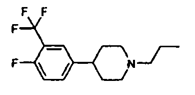 | + 94 |
| Example 43 of WO01/461 | |
| 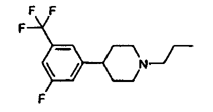 | + 239 |
| Example 44 of WO01/46146 | |
| 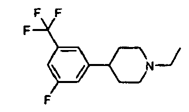 | + 232 |
| Claimed in WO01/46146 | |
| 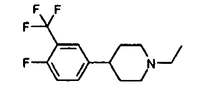 | + 75 |
| Claimed in WO01/46146 | |
| 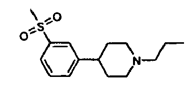 | + 114 |
| Example 6 of WO01/46145 | |
| | |

| **Examples** | **DOPAC** |
|---|---|
| | **%-increase** |
| 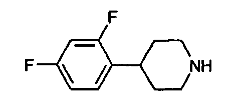 | - 36 |
| Example 1 | |
| 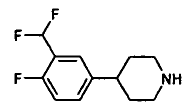 | + 17 |
| Example 2 | |
| 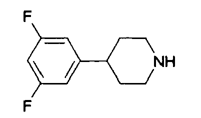 | - 19 |
| Example 3 | |
| 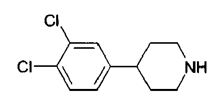 | - 14 |
| Example 6 | |
| 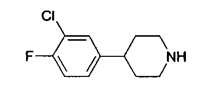 | - 29 |
| Example 9 | |
| 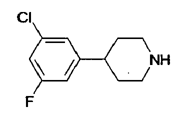 | - 26 |
| Example 10 | |

It can be clearly seen that - upon administration - the compounds described in WO01/46146 and WO01/4614 produce a significant increase in striatum DOPAC levels. In contrast, the compounds of the present invention have surprisingly been shown to provide a general decrease in striatum DOPAC levels, while certain compounds provide a negligible increase in striatum DOPAC levels. On the other hand, the essential characteristic of the compounds of the present invention is to produce increased cortical levels of catecholamines, measured as the extracellular levels of dopamine and norepinephrine assessed by the microdialysis technique, while displaying no or at most weak effects on subcortical catecholamines (FIGURES 11-22).

There is no guidance in the prior art how to obtain compounds that preferentially increase cortical catecholaminergic neurotransmission.

### Description of animal models used in the invention

The measurement of the tissue content of DOPAC is well established in the field of research since the 1960's. In short, male Sprague-Dawely rats are administered the test compound 60 minutes prior to decapitation. The brain is rapidly taken out and dissected. The striatum is rapidly frozen and subsequenlty quantitatively analysed with respect to its content of DOPAC by means of HPLC and electrochemical detection. The number of animals used for each test compound/vehicle is 4/group.

The microdialysis technique (Ungerstedt, Herrera-Marschitz et al. 1982) is a well established technique for measuring extracellular levels of neurotransmitters (Ungerstedt 1991). The microdialysis technique was used to measure the effect of drugs upon the monoamine transmitters. The appended graphs (Figures 9 and 10) show the effects of one established antidepressant (mirtazapine) upon monoamines in the striatum and frontal cortex, as well as for six compounds claimed in the present invention (Figures 11-24; 10 Examples 1, 3, 4, 6, 9, and 10). The number of animals (n) used for each compound tested is noted in the figure legend.

### Effects on dopamine and norepinephrine in cortical regions

### Cognition

The cortical circuitry underlying cognitive functions including memory, attention and working memory comprises a network of glutamatergic and GABAergic neurons, innervated by ascending dopaminergic and norepinephrinergic projections (Harrison and Weinberger 2005, Arnsten and Li 2005). Dopamine, acting through DA D1 receptors, enhances cognitive functions, while hypofunction of the cortical DA transmission produces specific cognitive deficits (reviewed in Goldman-Rakic, 2004). Likewise, norepinephrine has been found to enhance cognitive functions, presumably depending on stimulation of post-synaptic alpha-2 receptors in the prefrontal cortex (Arnsten, 2004). Clinical examples of the effects of cortical DA and NE deficiency are the cognitive disorders seen in schizophrenia and ADHD. In schizophrenia, cortical DA deficiency is regarded as a key feature underlying cognitive dysfunctions (Perlman et al, 2004, Goldman-Rakic, 2004). One mechanism by which such cortical DA hypofunction is believed to arise is a well described point mutation in the COMT encoding gene, leading to exagerrated activity of COMT, and therefore, an increased rate of elimination of DA, and ensuing, decreased levels of DA particularly in the cortex (Harrison and Weinberger 2005, Perlman et al, 2004). This mutation of COMT is genetically linked to schizophrenia as well as correlated to cognitive performance in healthy individuals. Apart from COMT anomalies, a variety of other pathogenetic pathways are proposed to lead to a functionally similar state of cortical dysfunction in schizophrenia, manifested by the characteristic abnormalities of cognitive functions seen in schizophrenic patients (Harrison and Weinberger, 2005). For instance, a number of susceptibility genes are thought to preferentially affect NMDA receptor mediated glutamate transmission. Due to the beneficial effects on cognitive functions by augmented DA D1 receptor stimulation, strengthening of cortical DA transmission can normalise cortical activity and enhance cognitive functions in schizophrenia as well as in other conditions (Goldman-Rakic, 2004). Furthermore, since the abnormalities in the cortical microcircuitry are regarded as the core feature underlying the clinical syndrome, restoration of this microcircuitry by facilitating DA transmission should not only improve cognitive functions in schizophrenia, but also reduce psychotic symptoms. Thus, normalisation of cortical DA transmission would as a secondary effect lead to normalisation of subcortical DA transmission, and thus, alleviation of the symptoms related to subcortical hyperdopaminergia (Goldman-Rakic, 2004, Perlman et al, 2004). Furthermore, a common feature of atypical antipsychotics, hypothesised to underlye their superior efficacy and fewer side effects compared to other antipsychotic compounds, is their ability to increase cortical dopamine (Moghaddam and Bunney, 1990, Deutch et al, 1991). It is important to note that the principle described in this invention to achieve cognitive enhancement and antipsychotic effects is dependent on regionally selective cortical increase in DA and NE, while increases in subcortical, eg striatal, DA are not sought for. In conclusion, compounds according to this invention, that increase cortical DA, but not subcortical DA transmission, will improve cognitive functions and reduce psychotic symptoms in schizophrenia.

The other clinical example showing the role of DA and NE in cognitive functions is the clinical features of ADHD, including the mode of action of compounds used to relieve the symptoms in this disorder. The key features of ADHD are deficiencies in attention, lack of ability to focus on a task for a prolonged time, impulsivity, and hyperactivity (Biederman 2005, Arnsten and Li 2005). In neuropsychological tests, ADHD patients perform poorly on tests specifically assessing prefrontal cortical functions (Arnsten and Li, 2005). The structure of the cortical circuitry underlying these functions suggests that insufficient DA and NE transmission would lead to the specific neuropsychological deficits seen in ADHD. Studies on the etiology of ADHD all point toward disregulation of DA and NE, particularly in cortical regions. The pharmacological treatments available are mainly psycho-stimulants, including dex-amphetamine, and methylphenidate, which increase DA and NE in most brain areas. A recent advancement in the treatment of ADHD is the compound atomoxetine (US 5,658,590), which produces regionally selective increases in cortical DA and NE, relieving core symptoms while avoiding side effects related to increase subcortical in DA transmission, thus supporting that cortical, rather than subcortical effects on catecholamines are essential to the clinical efficacy of ADHD medications (Pliszka, 2005).

Taken together, there is solid evidence that enhanced cortical DA and NE transmission would improve the symptoms of ADHD, including cognitive improvement. Furthermore, the role of cortical DA and NE in cognitive functions implies that enhancement of cortical DA transmission also improves cognitive functioning in cognitive disorders arising from causes other than schizophrenia or ADHD, as well as in healthy individuals. This is supported by the correlation between COMT activity and cognitive performance in healthy individuals (Perlman et al, 2004) and by numerous studies in rodents, primates and humans concerning the influence of cortical DA and NE on cognitive functions in healthy states as well as in different disorders (Arnsten, 2004, Goldman-Rakic, 2004). Consequently, the compounds according to the present invention will be useful to treat the symptoms of ADHD, as well as cognitive disorders in general, due to their ability to produce regionally selective increases in cortical DA and NE.

### Anxiolytic and antidepressant actions

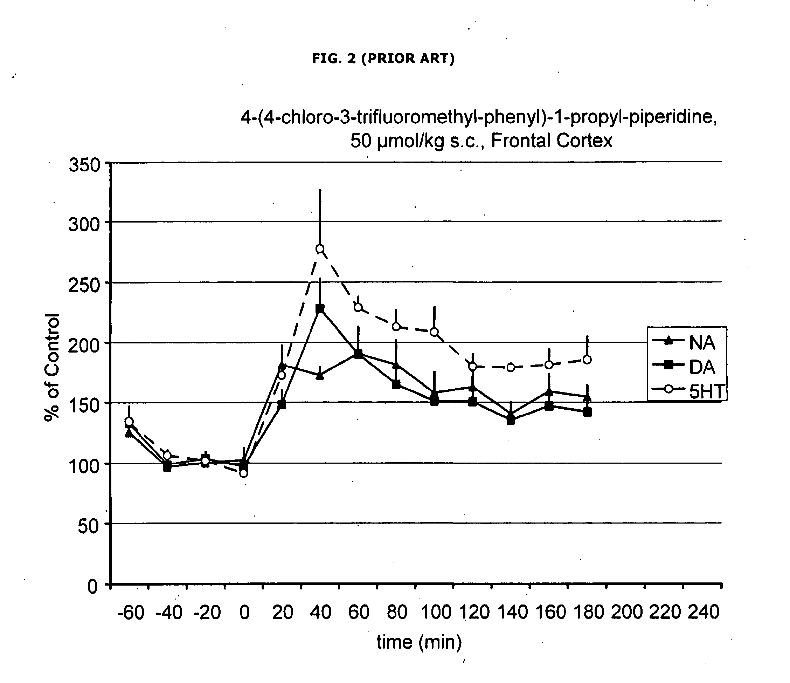

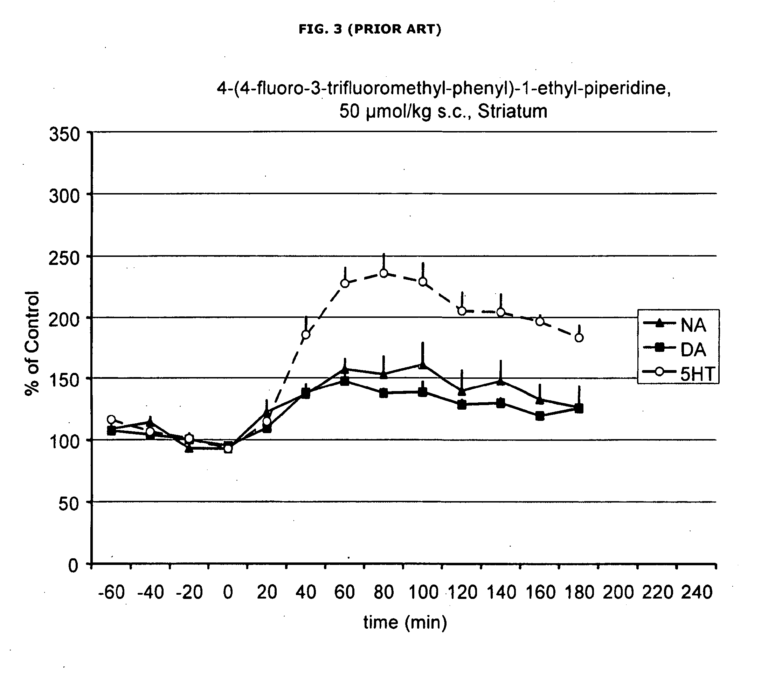

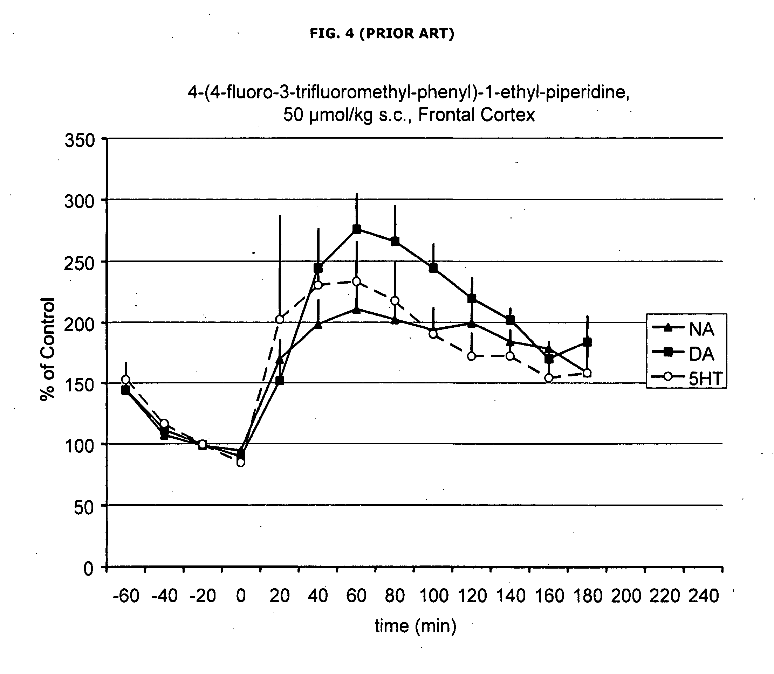

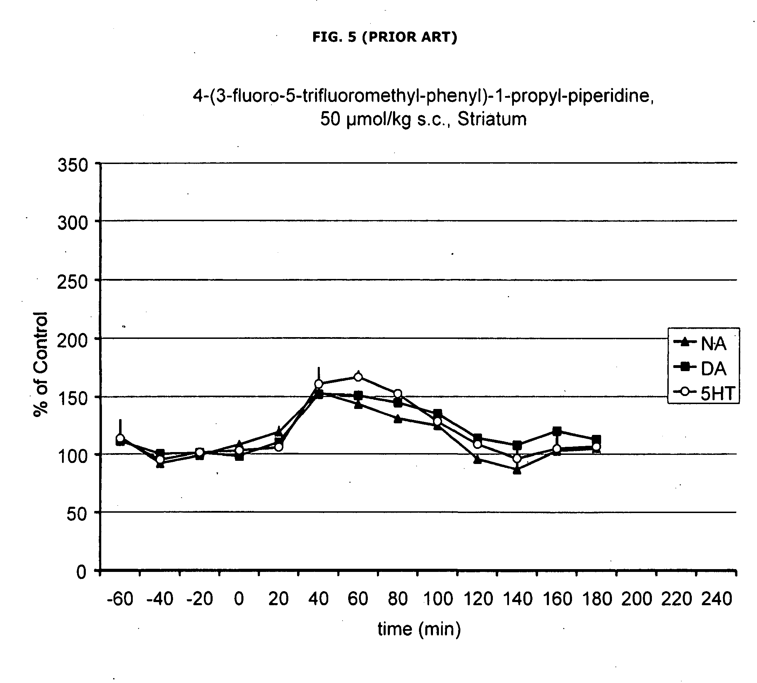

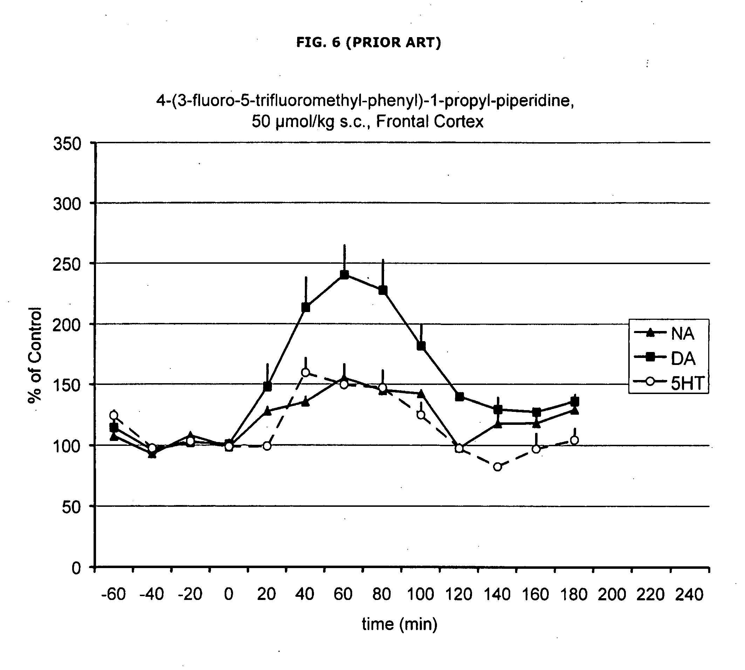

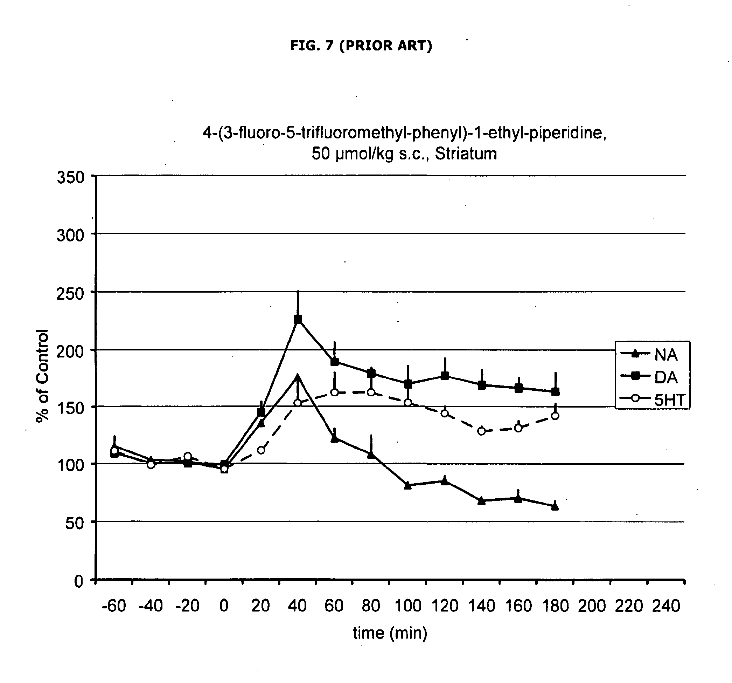

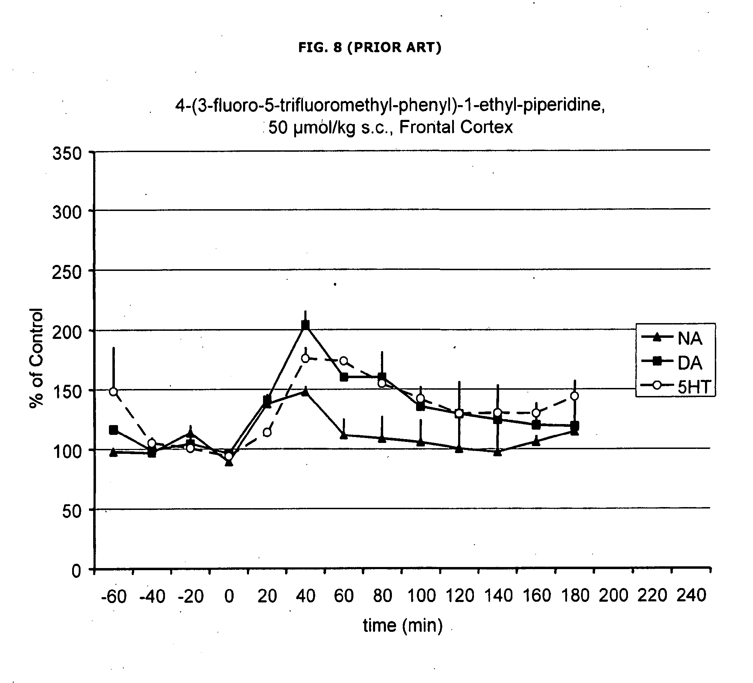

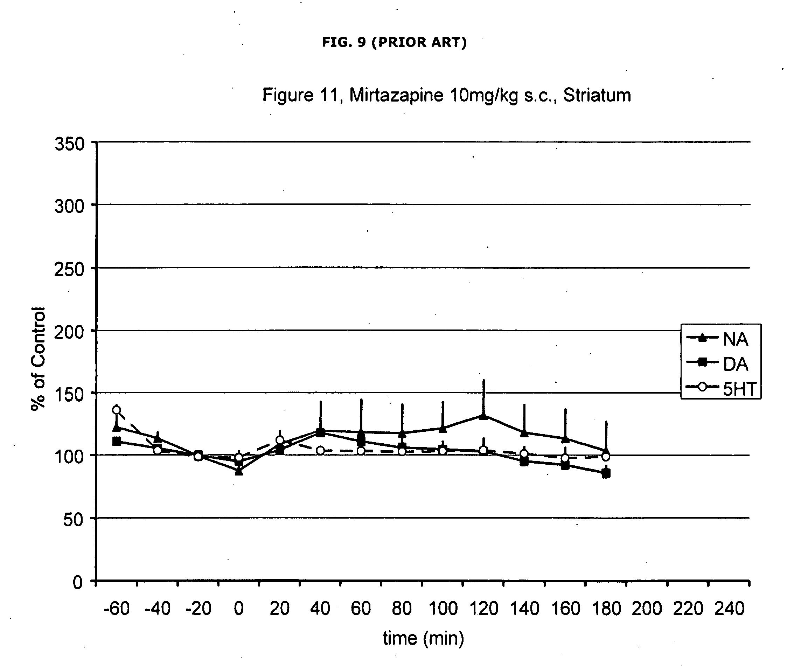

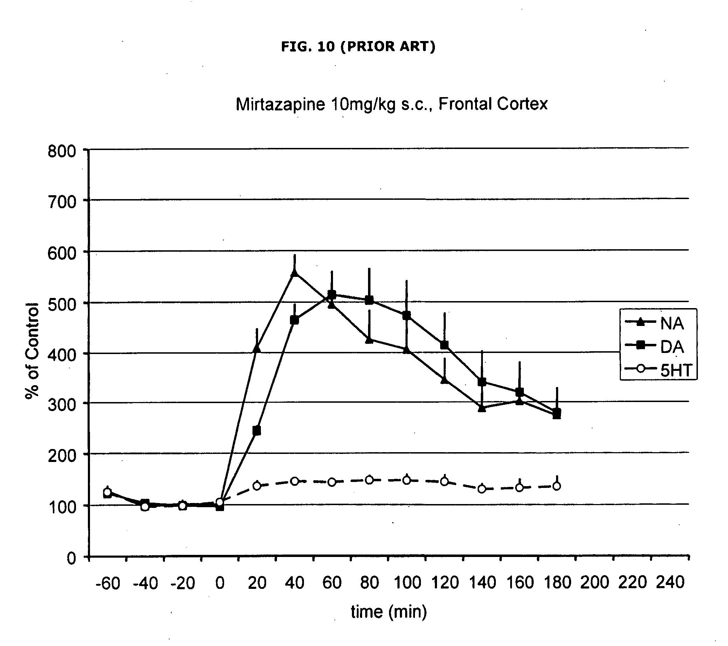

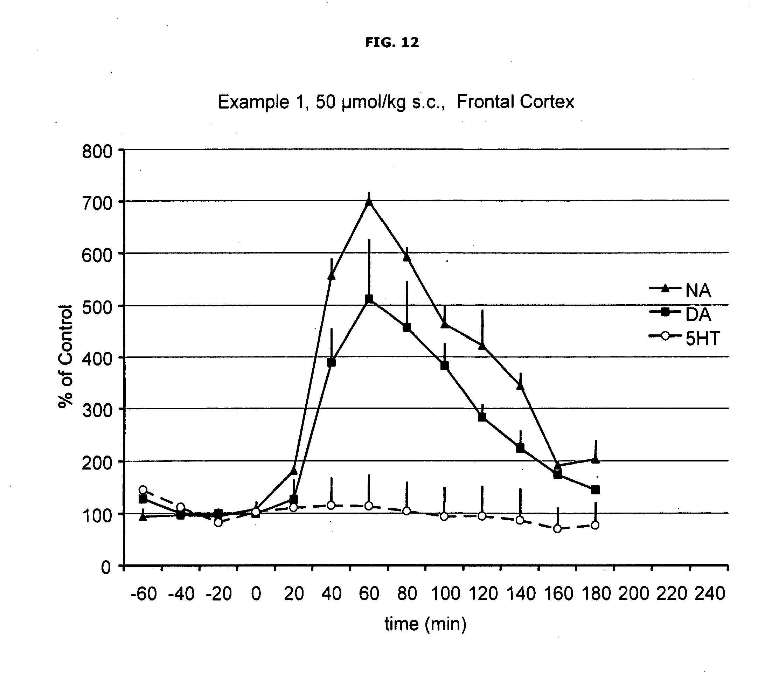

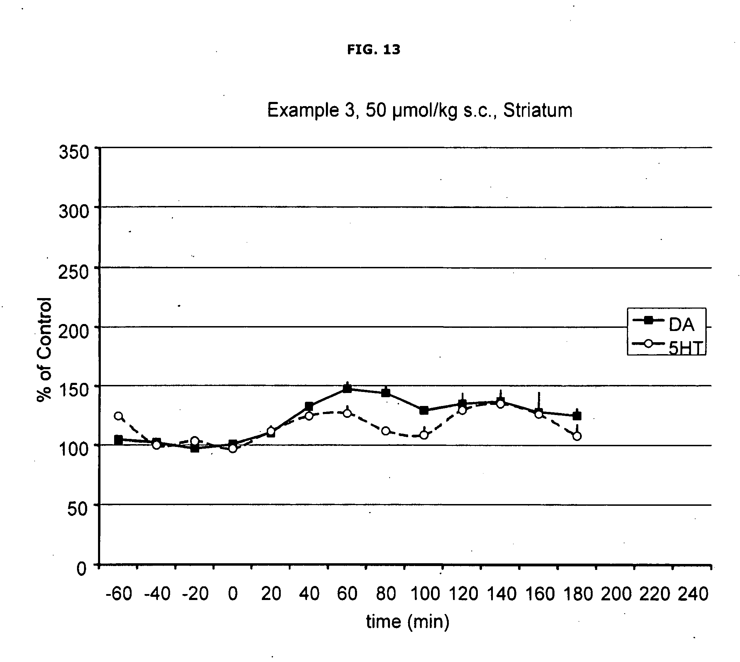

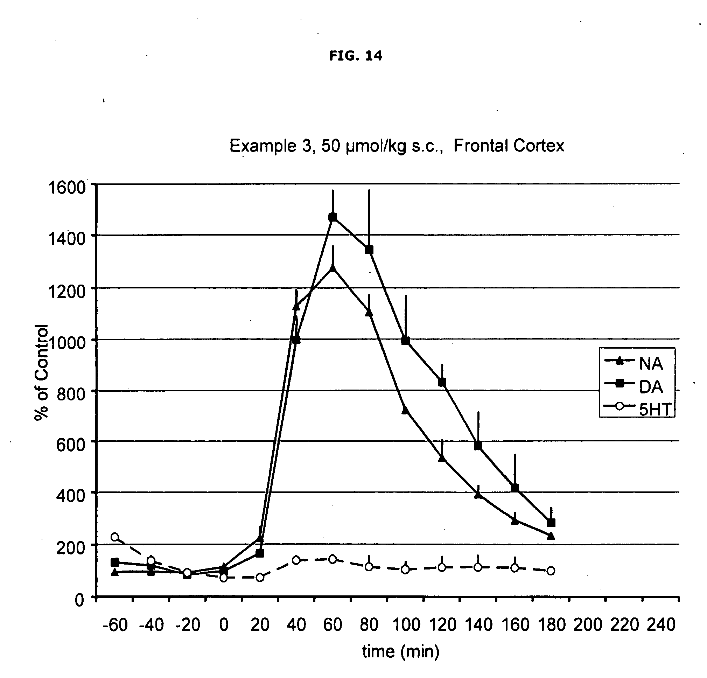

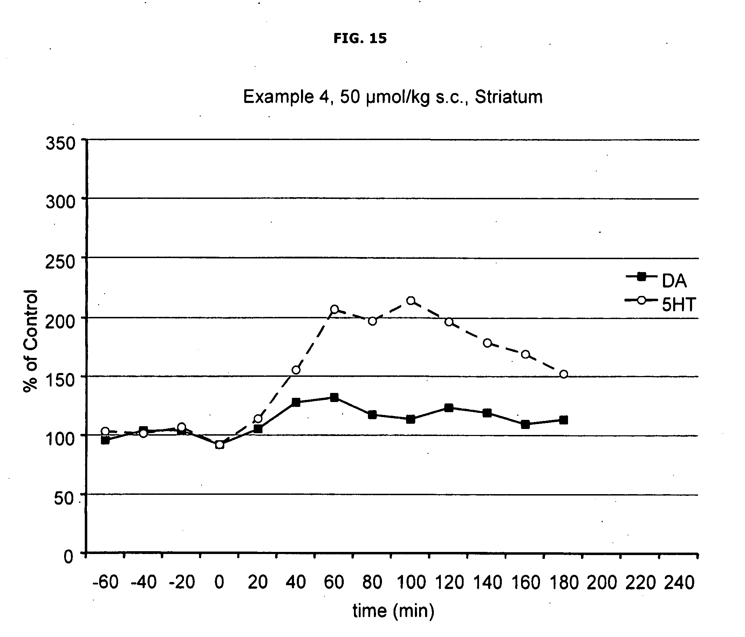

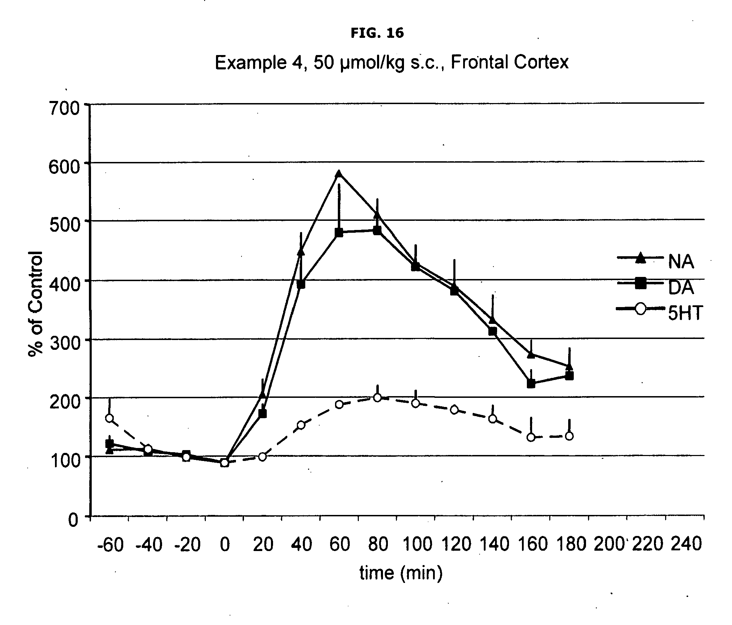

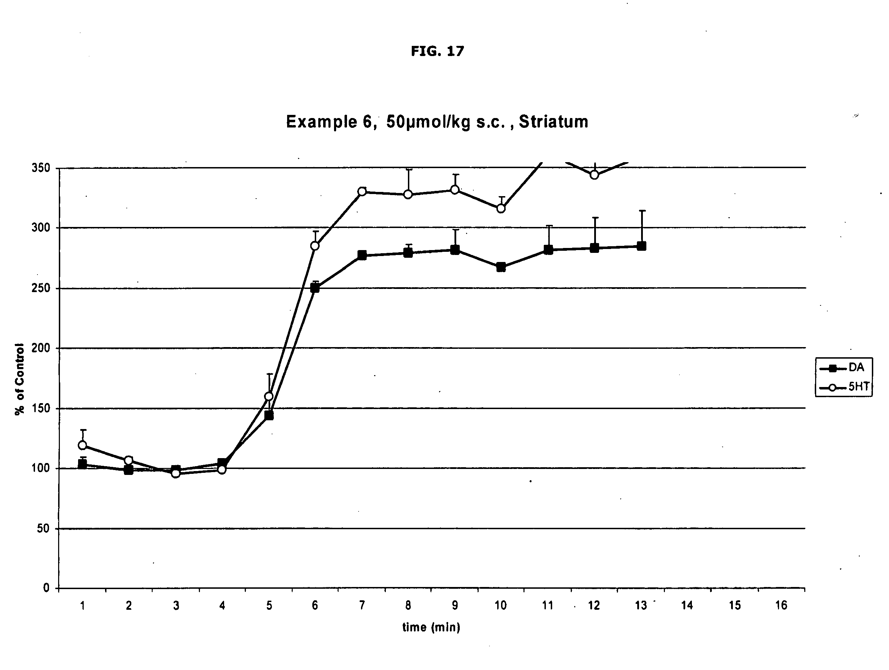

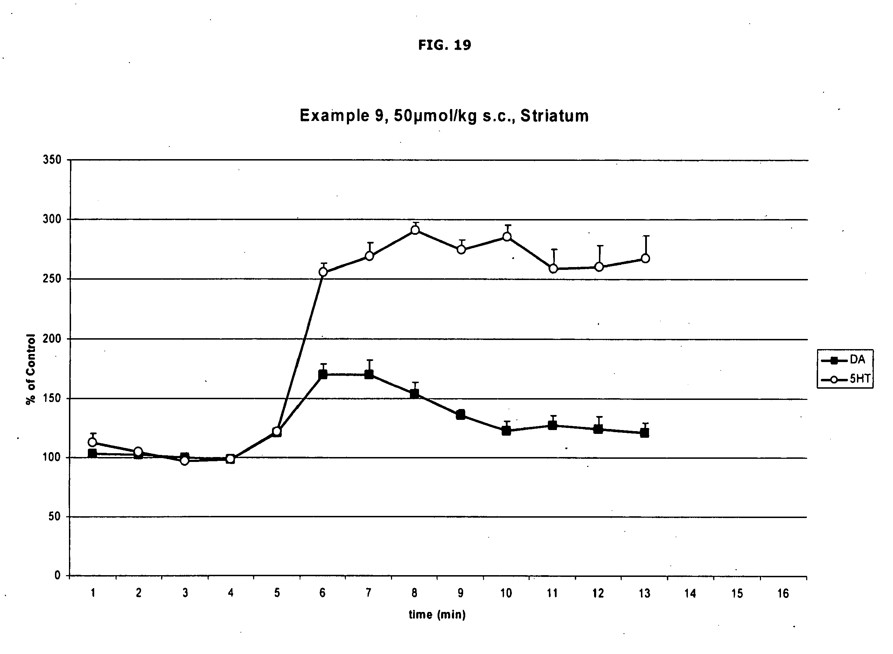

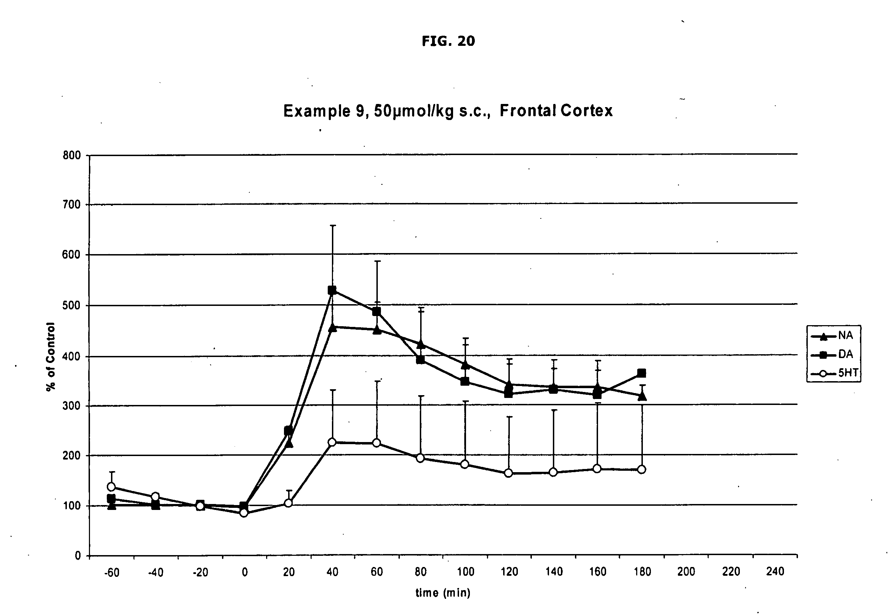

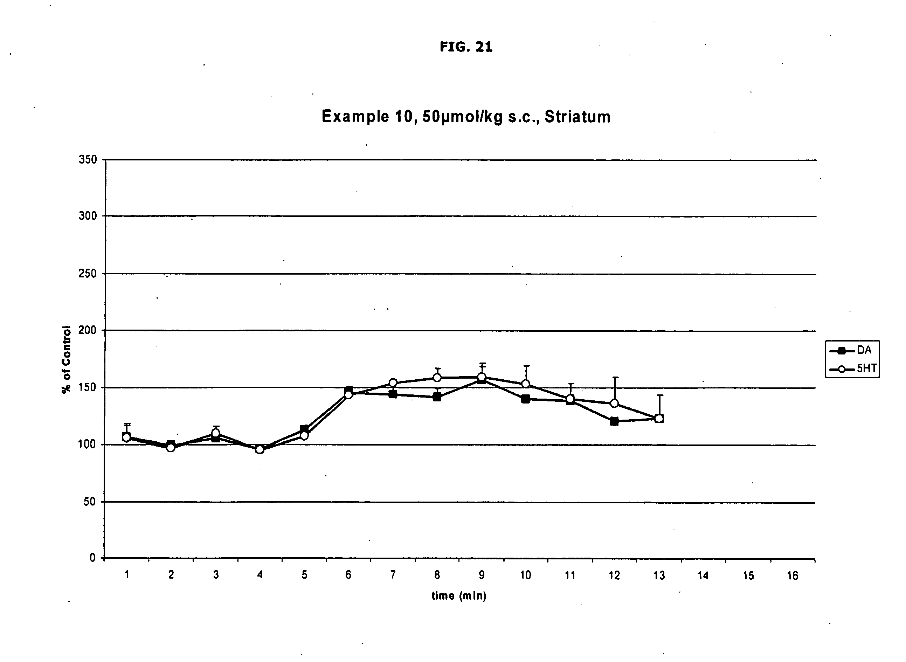

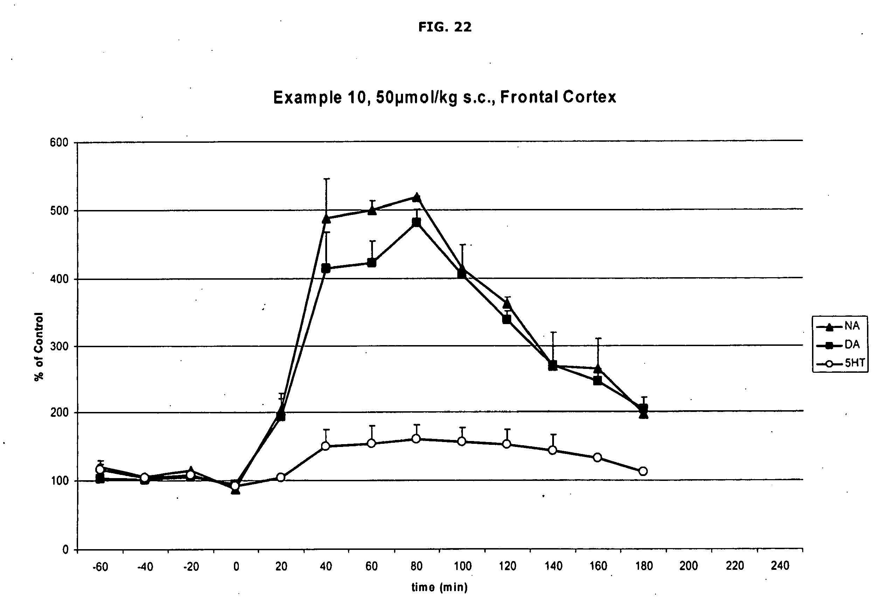

A common trait for all clinically effective classes of antidepressants is an elevation of the levels of dopamine and norepinephrine in the cortex (Tanda, Carboni et al. 1994; Millan, Lejeune et al. 2000). As an example, the clinically effective antidepressant mirtazapine (remeron) has been shown to increase predominantly extracelluilar-norepinephrine and dopamine in the cortex (See Figure 10, and Devoto, Flore et al. 2004). As the compounds claimed in the present invention elevate the levels of dopamine and norepinephrine in the cortex this supports our claim that they function as antidepressants (see Figures 11-24, Example 1, 3, 4, 6, 9, 10 and 11 in the present invention). Furthermore, norepinephrine is strongly involved in the neuronal pathways, comprising the locus ceruleus, the amygdala, and the cerebral cortex, controlling fear and anxiety and so, modulation of cortical norepinephrine transmission modulates states of anxiety (Sullivan et al 1999, Biol Psychiatry;46:1205-121). Accordingly, compounds that alters cortical norepinephrinergic transmission are reported to be effective in the treatment of anxiety disorders. More specifically, NE modulating compounds like mirtazapine (Remeron), which produces marked increases in cortical NE levels by a mechanism other than NE reuptake inhibition (Fig 10), and venlafaxine, which increases cortical NE by inhibition of norepinephrine re-uptake, both have anxiolytical properties in clinical studies (Neuropsychopharmacology, 5th generation of Progress, Lippincott, Williams and Wilkins 2002, pp 967 - 980). Based on this evidence for the beneficial effects of enhanced cortical norepinephrine transmission on anxiety disorders, along with the neurobiological back-ground demonstrating the crucial role of norepinephrine in the control of anxiety, it is concluded that the compounds of the present invention, which produces marked increases in cortical NE will be effective in the treatment of anxiety disorders.
Figure 1. 4-(4-chloro-3-trifluoromethyl-phenyl)-1-propyl-piperidine (Example 9 in WO01/46146) 50µmol/kg s.c. striatum amines
   4-(4-chloro-3-trifluoromethyl-phenyl)-1-propyl-piperidine is injected (s.c) at time-point 0. The values depicted in the graph represent percent of control in relation to baseline values. The microdialysis was performed in awake and freely moving rats. Dopamine = DA; Norepinephrine = NA; Serotonin = 5-HT; Error-bars=SEM
Figure 2 4-(4-chloro-3-trifluoromethyl-phenyl)-1-propyl-piperidine (Example 9 in WO01/46146) 50µmol/kg s.c. p.f. cortex
   4-(4-chloro-3-trifluoromethyl-phenyl)-1-propyl-piperidine is injected (s.c) at time-point 0. The values depicted in the graph represent percent of control in relation to baseline values. The microdialysis was performed in awake and freely moving rats. Dopamine = DA; Norepinephrine = NA; Serotonin = 5-HT Error-bars=SEM
Figure 3. 4-(4-fluoro-3-trifluoromethyl-phenyl)-1-ethyl-piperidine (claimed in WO01/46146) 50µmol/kg s.c. striatum amines
   4-(4-fluoro-3-trifluoromethyl-phenyl)-1-ethyl-piperidine is injected (s.c) at time-point 0. The values depicted in the graph represent percent of control in relation to baseline values. The microdialysis was performed in awake and freely moving rats. Dopamine = DA; Norepinephrine = NA; Serotonin = 5-HT; Error-bars=SEM
Figure 4. 4-(4-fluoro-3-trifluoromethyl-phenyl)-1-ethyl-piperidine (claimed in WO01/46146) 50µmol/kg s.c.. p.f. cortex
   4-(4-fluoro-3-trifluoromethyl-phenyl)-1-ethyl-piperidine is injected (s.c) at time-point 0. The values depicted in the graph represent percent of control in relation to baseline values. The microdialysis was performed in awake and freely moving rats. Dopamine = DA; Norepinephrine = NA; Serotonin = 5-HT; Error-bars=SEM
Figure 5. 4-(3-fluoro-5-trifluoromethyl-phenyl)-1-propyl-piperidine (Example 44 in WO01/46146) 50µmol/kg s.c. striatum amines
   4-(3-fluoro-5-trifluoromethyl-phenyl)-1-propyl-piperidine is injected (s.c) at time-point 0. The values depicted in the graph represent percent of control in relation to baseline values. The microdialysis was performed in awake and freely moving rats. Dopamine = DA; Norepinephrine = NA; Serotonin = 5-HT; Error-bars=SEM
Figure 6. 4-(3-fluoro-5-trifluoromethyl-phenyl)-1-propyl-piperidine (Example 44 in WO01/46146) 50µmol/kg s.c. p.f. cortex
   4-(3-fluoro-5-trifluoromethyl-phenyl)-1-propyl-piperidine is injected (s.c) at time-point 0. The values depicted in the graph represent percent of control in relation to baseline values. The microdialysis was performed in awake and freely moving rats. Dopamine = DA; Norepinephrine = NA; Serotonin = 5-HT; Error-bars=SEM
Figure 7. 4-(3-fluoro-5-trifluoromethyl-phenyl)-1-ethyl-piperidine (claimed in WO01/46146) 50µmol/kg s.c. striatum amines
   4-(3-fluoro-5-trifluoromethyl-phenyl)-1-ethyl-piperidine is injected (s.c) at time-point 0. The values depicted in the graph represent percent of control in relation to baseline values. The microdialysis was performed in awake and freely moving rats. Dopamine = DA; Norepinephrine = NA; Serotonin = 5-HT; Error-bars=SEM
Figure 8. 4-(3-fluoro-5-trifluoromethyl-phenyl)-1-ethyl-piperidine (claimed in WO01/46146) 50µmol/kg s.c.. p.f. cortex
   4-(3-fluoro-5-trifluoromethyl-phenyl)-1-ethyl-piperidine is injected (s.c) at time-point 0. The values depicted in the graph represent percent of control in relation to baseline values. The microdialysis was performed in awake and freely moving rats. Dopamine = DA; Norepinephrine = NA; Serotonin = 5-HT; Error-bars=SEM
Figure 9. Mirtazapine (Remeron) 10mg/kg s.c. p.f. cortex
   Remeron is injected (s.c.) at time-point 0. The values depicted in the graph represent percent of control in relation to baseline values. The microdialysis was performed in awake and freely moving rats. Dopamine = DA; Norepinephrine = NA; Serotonin = 5-HT; Error-bars=SEM
Figure 10. Mirtazapine (Remeron) 10mg/kg s.c. p.f. cortex
   Remeron is injected (s.c.) at time-point 0. The values depicted in the graph represent percent of control in relation to baseline values. The microdialysis was performed in awake and freely moving rats. Dopamine = DA; Norepinephrine = NA; Serotonin = 5-HT; Error-bars=SEM
Figure 11. Example 1, 50µmol/kg s.c. striatum amines n: 1-2
   Example 1 is injected (s.c.) at time-point 0. The values depicted in the graph represent percent of control in relation to baseline values. The microdialysis was performed in awake and freely moving rats. Dopamine = DA; Norepinephrine = NA; Serotonin = 5-HT; Error-bars=SEM
Figure 12. Example 1, 50µmol/kg s.c. pf. cortex amines n: 1-2
   Example 1 is injected (s.c.) at time-point 0. The values depicted in the graph represent percent of control in relation to baseline values. The microdialysis was performed in awake and freely moving rats. Dopamine = DA; Norepinephrine = NA; Serotonin = 5-HT; Error-bars=SEM
Figure 13. Example 3, 50µmol/kg s.c. striatum amines n: 1-2
   Example 3 is injected (s.c.) at time-point 0. The values depicted in the graph represent percent of control in relation to baseline values. The microdialysis was performed in awake and freely moving rats. Dopamine = DA; Norepinephrine = NA; Serotonin = 5-HT; Error-bars=SEM
Figure 14. Example 3, 50µmol/kg s.c. pf. cortex amines n: 1-2
   Example 3 is injected (s.c.) at time-point 0. The values depicted in the graph represent percent of control in relation to baseline values. The microdialysis was performed in awake and freely moving rats. Dopamine = DA; Norepinephrine = NA; Serotonin = 5-HT; Error-bars=SEM
Figure 15. Example 4, 50µmol/kg s.c. striatum amines n: 1-2
   Example 4 is injected (s.c.) at time-point 0. The values depicted in the graph represent percent of control in relation to baseline values. The microdialysis was performed in awake and freely moving rats. Dopamine = DA; Norepinephrine = NA; Serotonin = 5-HT; Error-bars=SEM
Figure 16. Example 4, 50µmol/kg s.c. pf. cortex amines n: 1-2
   Example 4 is injected (s.c.) at time-point 0. The values depicted in the graph represent percent of control in relation to baseline values. The microdialysis was performed in awake and freely moving rats. Dopamine = DA; Norepinephrine = NA; Serotonin = 5-HT; Error-bars=SEM
Figure 17. Example 6, 50µmol/kg s.c. striatum amines n: 1-2
   Example 6 is injected (s.c.) at time-point 0. The values depicted in the graph represent percent of control in relation to baseline values. The microdialysis was performed in awake and freely moving rats. Dopamine = DA; Norepinephrine = NA; Serotonin = 5-HT; Error-bars=SEM
Figure- 18. Example 6, 50µmol/kg s.c. pf. cortex amines n: 1-2
   Example 6 is injected (s.c.) at time-point 0. The values depicted in the graph represent percent of control in relation to baseline values. The microdialysis was performed in awake and freely moving rats. Dopamine = DA; Norepinephrine = NA; Serotonin = 5-HT; Error-bars=SEM
Figure 19. Example 9. 50µmol/kg s.c. striatum amines n: 1-2
   Example 9 is injected (s.c.) at time-point 0. The values depicted in the graph represent percent of control in relation to baseline values. The microdialysis was performed in awake and freely moving rats. Dopamine = DA; Norepinephrine = NA; Serotonin = 5-HT; Error-bars=SEM
Figure 20. Example 9, 50µmol/kg s.c. of. cortex amines n: 1-2
   Example 9 is injected (s.c.) at time-point 0. The values depicted in the graph represent percent of control in relation to baseline values. The microdialysis was performed in awake and freely moving rats. Dopamine = DA; Norepinephrine = NA; Serotonin = 5-HT; Error-bars=SEM
Figure 21. Example 10, 50µmol/kg s.c. striatum amines n: 1-2
   Example 10 is injected (s.c.) at time-point 0. The values depicted in the graph represent percent of control in relation to baseline values. The microdialysis was performed in awake and freely moving rats. Dopamine = DA; Norepinephrine = NA; Serotonin = 5-HT; Error-bars=SEM
Figure 22. Example 10, 50µmol/kg s.c. pf. cortex amines n: 1-2
   Example 10 is injected (s.c.) at time-point 0. The values depicted in the graph represent percent of control in relation to baseline values. The microdialysis was performed in awake and freely moving rats. Dopamine = DA; Norepinephrine = NA; Serotonin = 5-HT; Error-bars=SEM

### References:

Arnsten A.F.T. and Li B. (2005) Neurobiology of executive functions: Cathecholamine influences on prefrontal cortical functions BIOL PSYCHIATRY 2005;57:1377-1384 Biederman, J. Attention-Deficit/Hyperactivity Disorder: A selective overview BIOL PSYCHIATRY 2005;57:1215-1220
Harrison, P.J. and Weinberger, D.R. (2005) Schizophrenia genes, gene expression and neuropathology, on the matter of their convergence. Molecular Psychiatry 10: 40 - 68.
Moghaddam, B. and Bunney, B. S. (1990) Acute effects of typical and atypical antipsychotic drugs on the release of dopamine from prefrontal cortex, nucleus accumbens, and striatum of the rat: an in vivo microdialysis study. J. Neurochem 54, 5:1755-1759
Deutch AY, Moghaddam B, Innis RB, Krystal JH, Aghajanian GK, Bunney BS, Charney DS. (1991) Mechanisms of action of atypical antipsychotic drugs. Implications for novel therapeutic strategies for schizophrenia. Schizophr Res. Mar-Apr;4(2):121-56.
Pliszka, S.R. (2005) The neuropsychopharmacology of attention-deficit/hyperactivity disorder. Biol Psychiatry. 2005 Jun 1;57(11):1385-90. Review.
Ungerstedt, U. (1991). "Microdialysis-principles and applications for studies in animals and man." J. Int. Med. 230: 365-373.
Ungerstedt, U., M. Herrera-Marschitz, U. Jungnelius, L. Stahle, U. Tossman and T. Zetterström (1982). Dopamine Synaptic Mechanisms Reflected in Studies Combining Behavioural Recordings and Brain Dialysis. Advances in Dopamine Research. M. Kohksa. Oxford, Perganon Press. 37: 219-231.
Devoto, P., G. Flore, L. Pira, G. Longu and G. L. Gessa (2004). "Mirtazapine-induced corelease of dopamine and norepinephrine from noradrenergic neurons in the medial prefrontal and occipital cortex." Eur J Pharmacol 487(1-3): 105-11.
Millan, M. J., F. Lejeune and A. Gobert (2000). "Reciprocal autoreceptor and heteroreceptor control of serotonergic, dopaminergic and noradrenergic transmission in the frontal cortex: relevance to the actions of antidepressant agents." J Psychopharmacol 14(2): 114-38.
Tanda, G., E. Carboni, R. Frau and G. Di Chiara (1994). "Increase of extracellular dopamine in the prefrontal cortex: a trait of drugs with antidepressant potential?" Psychopharmacology (Berl) 115(1-2): 285-8.
Goldman-Rakic, P. et al (2004) Targeting the dopamine D1 receptor in schizophrenia: insights for cognitive dysfunction. Psychopharmacology 174:3-16
Arnsten, A. (2004) Adrenergic targets for the treatment of cognitive deficits in schizophrenia. Psychopharmacology 174:25-31

### METHODS OF PREPARATION

The compounds of the invention may be prepared as outlined below in Schemes 1-3. However, the invention is not limited to these methods. The compounds may also be prepared as described for structurally-related compounds in the prior art. The reactions can be carried out according to standard procedures^{1,2} or as described in the working examples. The starting materials for the processes described in the present application are known or may readily be prepared by conventional methods from commercially available chemicals.

Persons skilled in the art will appreciate that, in order to obtain compounds of the invention in an alternative - and in some occasions, more convenient manner - the individual process steps mentioned hereinbefore may be performed in a different order, and/or the individual reactions may be performed at different stage in the overall route (i.e. chemical transformations may be performed upon different intermediates to those associated hereinbefore with a particular reaction).

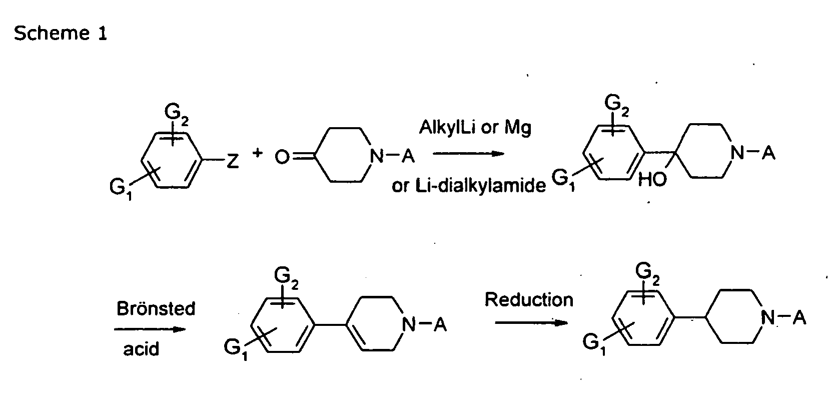

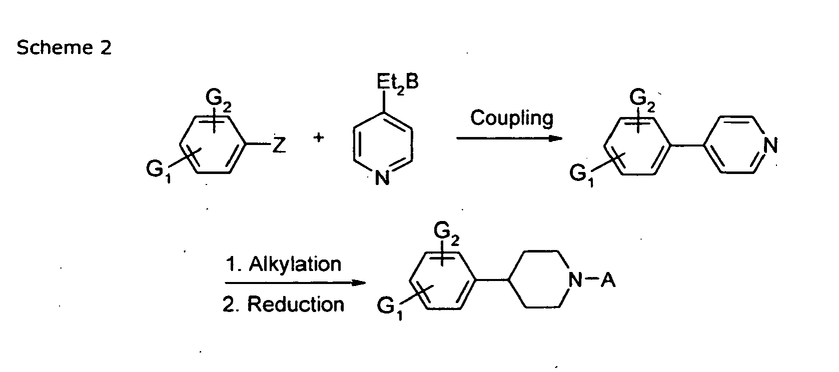

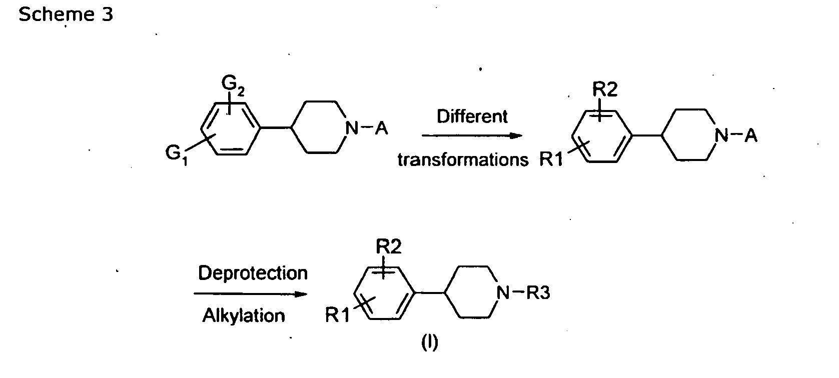

The substituents in Scheme 1-3, are as follows: Z is a leaving group, G1 is R1 or a group that can be transformed into R1, G2 is R2 or a group that can be transformed into R2, A is alkyl, hydrogen or a protecting group. X, R1, R2 and R3 are as defined above.

### References.

1. *Comprehensive Organic Transformations: A Guide to Functional Group Preparations* Richard C. Larock, 22 October, **1999** Wiley-VCH, ISBN: 0471190314
2. *March's Advanced Organic Chemistry: Reactions, Mechanisms, and Structure, 5th Edition.* Michael B. Smith, Jerry March, January 15, **2001** Wiley-Interscience, ISBN: 0471585890

The term "patient" used herein refers to an individual in need of the treatment according to the invention.

The term "treatment" used herein relates to both treatment in order to cure or alleviate a disease or a condition and to treatment in order to prevent the development of a disease or a condition. The treatment may either be performed in an acute or in a chronic way.

The compounds of the present invention may be isolated in any level of purity by standard methods and purification can be achieved by conventional means known to those skilled in the art, such as distillation, recrystallization and chromatography.

The present invention relates to pharmaceutical compositions comprising the compounds of the present invention, and their use in treating CNS disorders. Both organic and inorganic acids can be employed to form non-toxic pharmaceutically acceptable acid addition salts of the compounds according to the invention. Suitable acid addition salts of the compounds of the present invention include those formed with pharmaceutically acceptable salts such as toluensulfonate, methanesulfonate, fumarate, hydrochloride, hydrobromide, hydroiodide, nitrate, acetate, lactate, citrate, acid citrate, tartrate, bitartrate, aliphatic, alicyclic, aromatic or heterocyclic carboxylate, succinate, maleate, fumarate, gluconate, glycolate, saccharate, ascorbate, acetate, propionate, benzoate, pyruvate, pamoate [i.e., 1,1'-methylene-bis-(2-hydroxy-3-naphthoate)], phosphate, acid phosphate, sulphate or bisulfate salts. These salts are readily prepared by methods known in the art. It is also to be understood that compounds of the present invention can exist in solvated as well as unsolvated forms such as, e.g., hydrated forms.

The pharmaceutical composition comprising a compound according to the invention may also comprise substances used to facilitate the production of the pharmaceutical preparation or the administration of the preparations. Such substances are well known to people skilled in the art and may for instance be pharmaceutically acceptable adjuvants, carriers and preservatives.

In clinical practice, the compounds according to the present invention will normally be administered orally, rectally, nasally or by injection, in the form of pharmaceutical preparations comprising the active ingredient either as a free base or as a pharmaceutically acceptable non-toxic, acid addition salt, such as the hydrochloride, lactate, acetate or sulfamate salt, in association with a pharmaceutically acceptable carrier. The carrier may be a solid, semisolid or liquid preparation. Usually the active substance will constitute between 0.1 and 99% by weight of the preparation, more specifically between 0.5 and 20% by a weight for preparations intended for injection and between 0.2 and 50% by weight for preparations suitable for oral administration.

To produce pharmaceutical preparations containing the compound according to the invention in the form of dosage units for oral application, the selected compound may be mixed with a solid excipient, e.g. lactose, saccharose, sorbitol, mannitol, starches such as potato starch, corn starch or amylopectin, cellulose derivatives, a binder such as gelatine or polyvinyl-pyrrolidine, and a lubricant such as magnesium stearate, calcium stearate, polyethylene glycol, waxes, paraffin, and the like, and then compressed into tablets. If coated tablets are required, the cores (prepared as described above) may be coated with a concentrated sugar solution which may contain e.g. gum arabic, gelatine, talcum, titanium dioxide, and the like. Alternatively, the tablet can be coated with a polymer known to the man skilled in the art, dissolved in a readily volatile organic solvent or mixture of organic solvents. Dyestuffs may be added to these coatings in order to readily distinguish between tablets containing different active substances or different amounts of the active compound.

For the preparation of soft gelatine capsules, the active substance may be admixed with e.g. a vegetable oil or polyethylene glycol. Hard gelatine capsules may contain granules of the active substance using either the mentioned excipients for tablets e.g. lactose, saccharose, sorbitol, mannitol, starches (e.g. potato starch, corn starch or amylopectin), cellulose derivatives or gelatine. Also liquids or semisolids of the drug can be filled into hard gelatine capsules.

Examples of tablet and capsule formulations suitable for oral administration are given below:

| **Tablet I** | **mg/tablet** |
|---|---|
| Compound | 100 |
| Lactose Ph.Eur | 182.75 |
| Croscarmellose sodium | 12.0 |
| Maize starch paste (5% w/v paste) | 2.25 |
| Magnesium stearate | 3.0 |
| | |

| **Tablet II** | **mg/tablet** |
|---|---|
| Compound | 50 |
| Lactose Ph.Eur | 223.75 |
| Croscarmellose sodium | 6.0 |
| Maize starch | 15.0 |
| Polyvinylpyrrolidone (5% w/v paste) | 2.25 |
| Magnesium stearate | 3.0 |
| | |

| **Tablet III** | **mg/tablet** |
|---|---|
| Compound | 1.0 |
| Lactose Ph.Eur | 93.25 |
| Croscarmellose sodium | 4.0 |
| Maize starch paste (5% w/v paste). | 0.75 |
| Magnesium stearate | 1.0 |
| | |

| **Capsule** | **mg/capsule** |
|---|---|
| Compound | 10 |
| Lactose Ph.Eur | 488.5 |
| Magnesium | 1.5 |

Dosage units for rectal application can be solutions or suspensions or can be prepared in the form of suppositories comprising the active substance in a mixture with a neutral fatty base, or gelatine rectal capsules comprising the active substance in admixture with vegetable oil or paraffin oil. Liquid preparations for oral application may be in the form of syrups or suspensions, for example solutions containing from about 0.2% to about 20% by weight of the active substance herein described, the balance being sugar and mixture of ethanol, water, glycerol and propylene glycol. Optionally such liquid preparations may contain coloring agents, flavoring agents, saccharine and carboxymethylcellulose as a thickening agent or other excipients known to the man in the art.

Solutions for parenteral applications by injection can be prepared in an aqueous solution of a water-soluble pharmaceutically acceptable salt of the active substance, preferably in a concentration of from 0.5% to about 10% by weight. These solutions may also contain stabilizing agents and/or buffering agents and may conveniently be provided in various dosage unit ampoules. The use and administration to a patient to be treated would be readily apparent to an ordinary skill in the art.

For intranasal administration or administration by inhalation, the compounds of the present invention may be delivered in the form of a solution, dry powder or suspension. Administration may take place via a pump spray container that is squeezed or pumped by the patient or through an aerosol spray presentation from a pressurized container or a nebulizer, with the use of a suitable propellant, e.g., dichlorodifluoromethane, trichlorofluoromethane, dichlorotetrafluoroethane, carbon dioxide or other suitable gas. The compounds of the invention may also be administered via a dry powder inhaler, either as a finely divided powder in combination with a carrier substance (e.g. a saccharide) or as microspheres. The inhaler, pump spray or aerosol spray may be single or multi dose.

The dosage may be controlled through a valve that delivers a measured amount of active compound.

The compounds of the invention may also be administered in a controlled release formulation. The compounds are released at the required rate to maintain constant pharmacological activity for a desirable period of time. Such dosage forms provide a supply of a drug to the body during a predetermined period of time and thus maintain drug levels in the therapeutic range for longer periods of time than conventional non-controlled formulations. The compounds may also be formulated in controlled release formulations in which release of the active compound is targeted. For example, release of the compound may be limited to a specific region of the digestive system through the pH sensitivity of the formulation. Such formulations are well known to persons skilled in the art.

Depending upon the disorder and patient to be treated and the route of administration, the compositions may be administered at varying doses. The dosing will also depend upon the relation of potency to absorbability and the frequency and route of administration. Such doses may be administered once, twice or three or more times daily. The compounds of this invention can be administered to subjects in doses ranging from 0.01 mg to 500 mg per kg of body weight per day, although variations will necessarily occur depending upon the weight, sex and condition of the subject being treated, the disease state being treated and the particular route of administration chosen. However, a dosage level that is in the range of from 0.1 mg to 10 mg per kg of body weight per day, single or divided dosage is most desirably employed in humans for the treatment of diseases. Alternatively, the dosage level is such that a serum concentration of between 0.1 nM to 10 µM of the compound is obtained.

The invention is further illustrated in the examples below, which in no way are intended to limit the scope of the invention.

### Example 1:

### 4-(2,4-DIFLUOROPHENYL)PIPERIDINE

A mixture of 4-(2,4-difluorophenyl)-1,2,3,6-tetrahydropyridine (0.75 g, 3.8 mmol), palladium on carbon (0.21 g) and fumaric acid (0.75 ml) in isopropyl alcohol (20 ml) was hydrogenated at 50 psi for 15 h under hydrogen. The reaction mixture was filtered through a pad of celite and the filtrate was evaporated to dryness. Aqueous sodium carbonate (10%, 50 ml) and ethyl acetate (50 ml) was added and the phases were separated. The aqueous phase was extracted with ethyl acetate (2x50 ml) and the combined organic phases was dried (MgSO4) and evaporated under reduced pressure to give the pure product (0.39 g, 52%). The amine was converted to the hydrochloric acid salt and recrystallized from ethanol/diethyl ether: M.p. 256-258 °C. MS m/z (relative intensity, 70 eV) 197 (M+, bp), 196 (51), 140 (23), 127 (22), 56 (33).

### Example 2:

### 4-[3-(DIFLUOROMETHYL)-4-FLUOROPHENYL]PIPERIDINE

A mixture of 4-[3-(difluoromethyl)-4-fluorophenyl]pyridine (0.34 g, 1.52 mmol), platinum oxide (0.02 g) and hydrochloric acid (0.1 ml, conc) in methanol (10 ml) was hydrogenated at 50 psi for 15 h under hydrogen. The reaction mixture was filtered through a pad of celite and the filtrate was evaporated to dryness. Aqueous sodium carbonate (10%, 50 ml) was added. The aqueous phase was extracted with ethyl acetate (3x30 ml) and the combined organic phases was dried (MgS04), filtered and evaporated to dryness. Yield: 0.35 g. MS m/z (rel. intensity, 70 eV) 229 (M+, bp), 228 (64), 172 (12), 133 (9), 56 (32).

### Example 3:

### 4-(3,5-DIFLUOROPHENYL)PIPERIDINE

Preparation according to Example 2: 4-(3,5-difluorophenyl)-1,2,3,6-tetrahydropyridine (1.82 g, 9.33 mmol), platinum oxide (0.4 g), hydrochloric acid (0.1 ml, conc.), methanol (20 ml). Purification by flash chromatography (ethyl acetate/methanol 1:1) gave 0.85 g (46%) of the title compound. The amine was converted to the fumaric acid salt and recrystallized from ethanol/diisopropyl ether: M.p. 178 °C. MS m/z (relative intensity, 70 eV) 197 (M+, 87), 196 (46), 140 (20), 127 (28), 56 (bp).

### Example 4:

### 4-(3,4-DIFLUOROPHENYL)PIPERIDINE

A mixture of 4-(3,4-difluorophenyl)-1,2,3,6-tetrahydropyridine (7.5 g, 38.4 mmol) and palladium on carbon (1.36 g) in ethanol (30 ml) was hydrogenated at 50 psi for 15 h under hydrogen. The reaction mixture was filtered through a pad of celite and the filtrate was concentrated and evaporated to dryness to give 4.86 g of crude product. Purification by flash column chromatography (ethyl acetate/methanol 1:1) gave the title compound (2.3 g, 30%). The amine was converted to the hydrochloric acid salt and recrystallized from ethanol/diethyl ether: M.p. 255-256 °C. MS m/z (relative intensity, 70 eV) 197 (M+, 72), 196 (39), 140 (22), 127 (23), 56 (bp).

### Example 5:

### 4-[4-FLUORO-3-(TRIFLUOROMETHYL)PHENYL]PIPERIDINE

Preparation according to example 4: 4-[4-fluoro-3-(trifluoromethyl)phenyl]-1,2,3,6-tetrahydropyridine (0.4 g, 1.63 mmol), palladium on carbon (0.3, g) in ethanol (15 ml). Yield: 0.078 g (19%). The amine was converted to the hydrochloric acid salt and recrystallized from ethanol/diethyl ether: M.p. 195-196 °C. MS m/z (relative intensity, 70 eV) 247 (M+, 30), 246 (20), 190 (10), 169 (8), 56 (bp).

### Example 6:

### 4-(3,4-DICHLOROPHENYL)PIPERIDINE

Preparation according to Example 2: 4-(3,4-dichlorophenyl)-1,2,3,6-tetrahydropyridine (3.9. g, 17.1 mmol), platinum oxide (0.2 g), hydrochloric acid (0.1 ml, conc.), methanol (50 ml). Yield: 0.82 g (21%) of the title compound. The amine was converted to the hydrochloric acid salt and recrystallized from ethanol/diisopropyl ether: M.p. 170 °C. MS m/z (relative intensity, 70 eV) 231 (64), 230 (M+, 46), 2229 (bp), 228 (53), 56 (93).

### Example 7:

### 2-FLUORO-5-PIPERIDIN-4-YLBENZONITRILE

To a solution of 2-fluoro-5-piperidin-4-ylbenzamide (0.30 g, 1.35 mmol) in dry N,N-dimethylformamide (5 ml) was added freshly distilled phosphoryl trichloride (0.31 ml, 3.37 mmol) and the reaction mixture was stirred at 80°C for 1h. The solution was poured on to ice and was made basic by addition of aqueous sodium carbonate (10%, 50 ml). Ethyl acetate (50 ml) was added and the phases were separated. The aqueous phase was extracted with ethyl acetate (2x50 ml) and the combined organic phases was evaporated under reduced pressure to give an oil. The crude product was dissolved in a mixture of absolute ethanol (10 ml) and 3N hydrochloric acid (10 ml) and refluxed for 2 h. The ethanol was evaporated and the resulting aqueous mixture was made basic by addition of aqueous sodium carbonate (10%, 50 ml). Extraction with methylene chloride (2x50 ml) and evaporation under reduced pressure gave the pure product. Yield: 0.073 g (27%). The amine was converted to the hydrocloric acid salt and recrystallized from ethanol/diethyl ether. MS m/z (relative intensity, 70 eV) 204 (M+, bp), 203 (74), 205 (22), 198 (17), 56 (65).

### Example 8:

### 2-CHLORO-5-PIPERIDIN-4-YLBENZONITRILE

5-(1-benzylpiperidin-4-yl)-2-chlorobenzonitrile (0.12 g, 0.38 mmol) was dissolved in dry 1,2-dichloroethane (10 ml) and a-chloroethylchloroformat (0.041 ml, 0.38 mmol) was added. The resulting mixture was refluxed for 2 h, and the solvent was evaporated. The crude product was dissolved in methanol (20 ml) and the mixture was refluxed for 45 min. The solvent was evaporated and the crude product was purified on reversed phase preparative HPLC. Yield: 0.035 g (42%). The amine was converted to the hydrocloric acid salt and recrystallized from ethanol/diethyl ether. MS m/z (relative intensity, 70 eV) 222 (33), 221 (M+, 38), 220 (bp), 219 (79), 56 (80).

### Example 9:

### 4-(3-CHLORO-4-FLUOROPHENYL)PIPERIDINE

Preparation according to Example 2: 4-(3-chloro-4-fluorophenyl)-1,2,3,6-tetrahydropyridine (0.41 g, 1.94 mmol), platinum oxide (0.02 g), hydrochloric acid (0.1 ml, conc), methanol (20 ml). Yield: 0.21 g (50%) of the title compound. The amine was converted to the oxalic acid salt and recrystallized from ethanol/diethyl ether: M.p. 147 °C. MS m/z (relative intensity, 70 eV) 215 (32), 214 (M+, 29), 213 (bp), 212 (55), 56 (84).

### Example 10:

### 4-(3-CHLORO-5-FLUOROPHENYL)PIPERIDINE

Preparation according to Example 2: 4-(3-chloro-5-fluorophenyl)-1,2,3,6-tetrahydropyridine (1.5 g, 7.08 mmol), platinum oxide (0.4 g), hydrochloric acid (0.1 ml, conc), methanol (20 ml). Yield: 1.05 g (69%) of the title compound. The amine was converted to the hydrochloric acid salt and recrystallized from ethanol/diisopropyl ether: M.p. 243 °C. MS m/z (relative intensity, 70 eV) 214 (M+, 27), 213 (74), 212 (56), 178 (bp), 143 (22).

### Example 12:

### 4-[4-CHLORO-3-(TRIFLUOROMETHYL)PHENYL]PIPERIDINE

Preparation according to Example 2: 4-[4-chloro-3-(trifluoromethyl)phenyl]-1,2,3,6-tetrahydropyridine (0.5 g, 1.91 mmol), platinum oxide (0.02 g), hydrochloric acid (0.1 ml, conc), methanol (20 ml). Yield: 0.33 g (65%) of the title compound. MS m/z (relative intensity, 70 eV) 265 (24), 264 (M+, 27), 263 (M+, 75), 262 (48), 56 (bp).

### Example 13:

### 4-(2-CHLORO-4-FLUOROPHENYL)PIPERIDINE

A mixture of 4-(2-chloro-4-fluorophenyl)pyridine (1.16 g, 5.59 mmol) and platinum oxide (0.1 g) in methanol (40 ml) was hydrogenated at 50 psi for 48 h under hydrogen. The reaction mixture was filtered through a pad of celite and the filtrate was evaporated to dryness. The crude product was purified on reversed phase preparative HPLC. Yield: 0.5 g (42%). MS m/z (rel. intensity, 70 eV) 214 (29), 213 (M+, 75), 212 (62), 178 (bp), 56 (99).

The following Preparations are used in the synthesis of the above Examples.

### Preparation 1:

### TERT-BUTYL 4-(2,4-DIFLUOROPHENYL)-4-HYDROXYPIPERIDINE-1-CARBOXYLATE

To a solution of 1-bromo-2,4-difluorobenzene (5.0 g, 25.9 mmol) in dry diethyl ether (70 ml), under nitrogen, was added dropwise at -78°C, hexyllithium (2.3 M in hexane, 11.2 ml, 25.9 mmol). The mixture was stirred for 1 h after which a solution of 4-boc-1-piperidone (5.1 g, 25.9 mmol) in dry diethyl ether (50 ml) was added drop wise. The resulting mixture was stirred at -78°C for 30 min and then brought to ambient temperature. Water (100 ml) was added and the mixture was extracted with ethyl acetate (3x100 ml). The combined organic phases was dried (MgSO4), filtered and evaporated to dryness. The oily residue was purified by flash column chromatography (ethyl acetate/isooctane, 1:1) to give the title compound (4.3 g). MS m/z (rel. intensity, 70 eV) 313 (M+, 4), 239 (90),195 (34), 141 (31), 57 (bp).

### Preparation 2:

### 4-(2,4-DIFLUOROPHENYL)-1,2,3,6-TETRAHYDROPYRIDINE

To a solution of tert-butyl-4-(2,4-difluorophenyl)-4-hydroxypiperidine-1-carboxylate (4.32 g, 13.8 mmol) in toluene (100 ml) was added sulfuric acid (18M, 0.95 ml, 17.1 mmol) and the mixture was refluxed under a Dean-Stark water separator for 2 hours. The mixture was poured on to ice and was basified with aqueous sodium hydroxide (15%). The mixture was extracted with ethyl acetate (3x50 ml) and the combined organic phases was dried (MgSO4), filtered and evaporated to dryness to yield the crude product (2.16 g). MS m/z (rel. intensity, 70 eV) 195 (M+, bp), 194 (41), 165 (21), 151 (24), 127 (47).

### Preparation 3:

### 4-BROMO-2-(DIFLUOROMETHYL)-1-FLUOROBENZENE

5-bromo-2-fluorobenzaldehyde (2.0 g, 9.85 mmol) and diethylaminosulfur trifluoride (2.2 ml) was stirred at ambient temperature for 1 hour. Methylene chloride (100 ml) was added and the solution was cooled to 0 °C. Aqueous sodium bicarbonate (10%, 50 ml) was added slowly and the phases were separated. The organic phase was washed with water (50 ml), dried (MgSO4), filtered and evaporated to dryness to yield the crude product (1.79 g). MS m/z (rel: intensity, 70 eV) 226 (M+, 98), 224 (M+, bp), 207 (17), 145 (79), 125 (22).

### Preparation 4:

### 4-[3-(DIFLUOROMETHYL)-4-FLUOROPHENYL]PYRIDINE

To a mixture of 4-bromo-2-(difluoromethyl)-1-fluorobenzene (0.5 g, 2.24 mmol), 1-pyridyl-4-boronic acid (0.33 g, 2.69 mmol) and sodium carbonate (0.64 g, 5.6 mmol) in toluene/ethanol (1:1, 20 ml) under nitrogen, was added palladium tetrakis (0.13 g , 5 mol%). The mixture was heated at reflux for 16 h, cooled to ambient temperature and diluted with water (50 ml) and ethyl acetate (100 ml). The organic layer was separated and the aqueous phase was extracted with ethyl acetate (2x50 ml). The combined organic phases was evaporated to dryness and dissolved in aqueous hydrochloric acid (10%, 50 ml). The solution was washed with diethyl ether (2x40 ml), basified with aqueous sodium hydroxide (2M) and extracted with ethyl acetate (2x50 ml). The combined organic phases was dried (MgSO4) and evaporated to dryness to give the crude product. Purification by flash column chromatography (ethyl acetate/isooctane 2: 1) gave the title compound (0.34 g). MS m/z (rel. intensity, 70 eV) 223 (M+, bp), 222 (12), 204 (7), 172 (12), 145 (8).

### Preparation 5:

### TERT-BUTYL 4-(3,5-DIFLUOROPHENYL)-4-HYDROXYPIPERIDINE-1-CARBOXYLATE

Preparation according to preparation 1: 1-bromo-3,5-difluorobenzene (7.0 g, 36.2 mmol), n-butyl lithium (2.5 M in hexane, 18.7 ml, 36.2 mmol), 4-boc-1-piperidone (7.9 g, 39.8 mmol), tetrahydrofuran (150 ml). Yield: 10.6 g. MS m/z (rel. intensity, 70 eV) 313 (M+, 2), 239 (40), 195 (16), 141 (18), 57 (bp).

### Preparation 6:

### 4-(3,5-DIFLUOROPHENYL)-1,2,3,6-TETRAHYDROPYRIDINE

A solution of tert-butyl 4-(3,5-difluorophenyl)-4-hydroxypiperidine-1-carboxylate (7.8 g, 24.9 mmol) in trifluoroacetic acid (30 ml) was heated at reflux for 20 h. The mixture was poured on to ice and was basified with aqueous sodium hydroxide (10M). The mixture was extracted with ethyl acetate (3x100 ml) and the combined organic phases was dried (MgS04), filtered and evaporated to dryness to give the title compound (7.6 g). MS m/z (rel. intensity, 70 eV) 195 (M+, 53), 138 (45), 136 (bp), 127 (78), 57 (76).

### Preparation 7:

### TERT-BUTYL 4-(3,4-DIFLUOROPHENYL)-4-HYDROXYPIPERIDINE-1-CARBOXYLATE

Preparation according to preparation 1: 1-bromo-3,4-difluorobenzene (7.0 g, 36.2 mmol), tetrahydrofuran (100 ml), n-butyl lithium (2.5 M in hexane, 18.7 ml, 36.2 mmol), 4-boc-1-piperidone (7.9 g, 39.8 mmol). Yield: 8.7 g. MS m/z (rel. intensity, 70 eV) 313 (M+, 2), 239 (28),195 (14), 141 (13), 57 (bp).

### Preparation 8:

### 4-(3,4-DIFLUOROPHENYL)-1,2,3,6-TETRAHYDROPYRIDINE

Preparation according to preparation 6: tert-butyl 4-(3,4-difluorophenyl)-4-hydroxypiperidine-1-carboxylate (8.7 g, 27.8 mmol), trifluoroacetic acid (40 ml). Yield: 7.5 g. MS m/z (rel. intensity, 70 eV) 195 (M+, 53), 138 (45), 136 (bp), 127 (78), 57 (76).

### Preparation 9:

### TERT-BUTYL-4-[4-FLUORO-3-(TRIFLUOROMETHYL)PHENYL]-4-HYDROXYPIPERIDINE-1-CARBOXYLATE

Preparation according to preparation 5: 4-bromo-1-fluoro-2-(trifluoromethyl)benzene (5.0 g, 20.5 mmol), tetrahydrofuran (100 ml), n-butyl lithium (2.5 M in hexane, 8.2 ml, 20.5 mmol), 4-boc-1-piperidone (4.9 g, 24.6 mmol). Yield: 5.05 g. MS m/z (rel. intensity, 70 eV) 363 (M+, 2), 290 (17), 289 (18), 191 (9), 57 (bp).

### Preparation 10:

### 4-[4-FLUORO-3-(TRIFLUOROMETHYL)PHENYL]-1,2,3,6-TETRAHYDROPYRIDINE

Preparation according to preparation 6: tert-butyl-4-[4-fluoro-3-(trifluoromethyl)phenyl]-4-hydroxypiperidine-1-carboxylate (4.25 g, 11.7 mmol), trifluoroacetic acid (40 ml). Yield: 1.28 g. MS m/z (rel. intensity, 70 eV) 263 (M+, 9), 245 (59), 244 (29), 163 (20), 56 (bp).

### Preparation 11:

### TERT-BUTYL 4-(3,4-DICHLOROPHENYL)-4-HYDROXYPIPERIDINE-1-CARBOXYLATE

To a mixture of magnesium turnings (0.59 g, 24.3 mmol), activated with iodine in dry diethyl ether (5 ml), under nitrogen, was added dropwise, a solution of 4-bromo-1,2-dichlorobenzene (5.0 g, 22.1 mmol) in dry diethyl ether (20 ml). The mixture was heated at reflux for 0.5 h after which a solution of 4-Boc-1-piperidone (4.4 g, 22.1 mmol) in dry diethyl ether (20 ml) was added dropwise. The reaction mixture was stirred for 45 minutes after which saturated aqueous ammonium chloride (100 ml) was added. The residue was extracted with ethyl acetate (3x50 ml) and the combined organic phases was dried (MgS04), filtered and evaporated to dryness. The oily residue was purified by flash column chromatography (isooctane/ethyl acetate, 1:1) to give the title compound (5.1 g). MS m/z (rel. intensity, 70 eV) 346 (M+, 1), 245 (42), 229(64), 227 (bp), 226 (51).

### Preparation 12:

### 4-(3,4-DICHLOROPHENYL)-1,2,3,6-TETRAHYDROPYRIDINE

Preparation according to preparation 6: tert-butyl 4-(3,4-dichlorophenyl)-4-hydroxypiperidine-1-carboxylate (5.1 g, 14.7 mmol), trifluoroacetic acid (10 ml). Yield: 4.0 g. MS m/z (rel. intensity, 70 eV) 229 (65), 228 (M+, 53), 227 (bp), 226 (68), 163 (56).

### Preparation 13:

### 5-BROMO-2-FLUOROBENZAMIDE

To a solution of 5-bromo-2-fluorobenzoic acid 1.0 g, 4.56 mmol) in methylene chloride (20 ml) was added thionyl chloride (2 ml, 27.3 mmol). The reaction mixture was stirred at 55°C for 2 h and the solvents were evaporated. The crude product was dissolved in methylene chloride (20 ml) and quenched with aqueous ammonium hydroxide (5 ml, 32%). After an additional hour of stirring, water (100 ml) was added and the organic phase was collected. The aqueous phase was extracted with methylene chloride (50 ml) and the pooled organic phase was dried dried (MgSO4), filtered and evaporated to dryness. Yield: 0.68 g. MS m/z (rel. intensity, 70 eV) 218 (M+, 73), 217 (75), 201 (bp), 175 (31), 94 (64).

### Preparation 14:

### 2-FLUORO-5-PYRIDIN-4-YLBENZAMIDE

To a mixture of 5-bromo-2-fluorobenzamide (0.68 g, 3.11 mmol), 1-pyridyl-4-boronic acid (0.46 g, 3.73 mmol) and sodium carbonate (0.92 g, 8.7 mmol) in toluene/ethanol (1:1, 30 ml) under nitrogen, was added palladium tetrakis (0.18 g, 5 mol%). The mixture was heated at reflux for 15 h, cooled to ambient temperature and diluted with water (50 ml) and ethyl acetate (100 ml). The organic layer was separated and the aqueous phase was extracted with ethyl acetate (2x50 ml). The combined organic phases was evaporated to dryness and dissolved in aqueous hydrochloric acid (10%, 50 ml). The solution was washed with diethyl ether (2x40 ml), basified with aqueous sodium hydroxide (2M) and extracted with ethyl acetate (2x50 ml). The combined organic phases was dried (MgSO₄) and evaporated to dryness to give the title compound (0.58 g). MS m/z (rel. intensity, 70 eV) 216 (M+, 79), 201 (14), 200 (bp), 172 (22), 125 (13).

### Preparation 15:

### 2-FLUORO-5-PIPERIDIN-4-YLBENZAMIDE

To a solution of 2-fluoro-5-pyridin-4-ylbenzamide (0.58 g, 2.7 mmol) in methanol (20 ml), was added platinum oxide (0.02 g) and hydrochloric acid (0.1 ml, conc.) and the reaction mixture was hydrogenated at 50 psi for 15 h under hydrogen. Filtration through a pad of celite and evaporation of the filtrate gave 0.48 g of crude product as the hydrochloric acid salt. The crude product was purified by reversed phase preparative HPLC to give the title compound (0.30 g). MS m/z (relative intensity, 70 eV) 223 (45), 222 (M+, bp), 221 (74), 149(32), 57 (87).

### Preparation 16:

### 5-BROMO-2-CHLOROBENZAMIDE

Preparation according to preparation 13: 5-bromo-2-chlorobenzoic acid 4.0 g, 17.0 mmol), methylene chloride (100 ml), thionyl chloride (6 ml, 81.9 mmol), ammonium hydroxide (20 ml, 32%). Yield: 3.2 g. MS m/z (rel. intensity, 70 eV) 235 (M+, 50), 233 (M+, 39), 219 (bp), 217 (78), 75 (38).

### Preparation 17:

### 2-CHLORO-5-PYRIDIN-4-YLBENZAMIDE

Preparation according to preparation 14: 5-bromo-2-chlorobenzamide (3.2 g, 13.6 mmol), 1-pyridyl-4-boronic acid (2.01 g, 16.4 mmol), sodium carbonate (3.62 g, 34.2 mmol), palladium tetrakis (0.79 g, 5 mol%), toluene/ethanol (1:1, 50 ml). Yield (1.6 g). MS m/z (rel. intensity, 70 eV) 234 (M+, 25), 232 (M+, 74), 218 (33), 216 (bp), 153 (19).

### Preparation 18:

### 2-CHLORO-5-PIPERIDIN-4-YLBENZAMIDE

Preparation according to preparation 15: 2-chloro-5-pyridin-4-ylbenzamide (1.58 g, 6.8 mmol), platinum oxide (0.08 g), hydrochloric acid (0.1 ml, conc.), methanol (20 ml). Yield: 0.90 g

### Preparation 19:

### 5-(1-BENZYLPIPERIDIN-4-YL)-2-CHLOROBENZAMIDE

To a solution of 2-chloro-5-piperidin-4-ylbenzamide (0.66 g, 2.76 mmol) in acetonitrile (20 ml) was added potassium carbonate (0.53 g, 3.86 mmol) and benzyl bromide (0.328 ml, 2.76 mmol) and the mixture was stirred for 6 h. Water (50 ml) was added, the aqueous residue was extracted with ethyl acetate (3x50 ml) and the combined organic phases was dried and concentrated to give the title compound (0.25 g). MS m/z (relative intensity, 70 eV) 329 (M+, 16), 328 (26), 327 (34), 207 (30) 91 (bp).

### Preparation 20:

### 5-(1-BENZYLPIPERIDIN-4-YL)-2-CHLOROBENZONITRILE

Preparation according to exampel 7: 5-(1-benzylpiperidin-4-yl)-2-chlorobenzamide (0.25 g, 0.76 mmol), dry N,N-dimethylformamide (5 ml), phosphoryl trichloride (0.17 ml, 1.9 mmol. Yield: 0.12 g. MS m/z (relative intensity, 70 eV) 311 (M+, 15), 310 (34), 309 (28), 219 (29), 91 (bp).

### Preparation 21:

### BENZYL 4-(3-CHLORO-4-FLUOROPHENYL)-4-HYDROXYPIPERIDINE-1-CARBOXYLATE

Preparation according to preparation 1: 4-bromo-2-chloro-1-fluorobenzene (7.5 g, 35.9 mmol), diethyl ether (100 ml), n-butyl lithium (2.5 M in hexane, 9.6 ml, 23.9 mmol), 4-cbz-1-piperidone (6.1 g, 26.3 mmol). Yield: 5.7 g. MS m/z (rel. intensity, 70 eV) 363 (M+, 1), 241 (6), 157 (5), 92 (9), 91 (bp).

### Preparation 22:

### 4-(3-CHLORO-4-FLUOROPHENYL)-1,2,3,6-TETRAHYDROPYRIDINE

Preparation according to preparation 6: benzyl 4-(3-chloro-4-fluorophenyl)-4-hydroxy-piperidine-1-carboxylate (5.7 g, 15.7 mmol), trifluoroacetic acid (25 ml). Yield: 1.05 g. MS m/z (rel. intensity, 70 eV) 211 (M+, bp), 210 (63), 147 (66), 146 (39), 82 (37).

### Preparation 23:

### TERT-BUTYL 4-(3-CHLORO-5-FLUOROPHENYL)-4-HYDROXYPIPERIDINE-1-CARBOXYLATE

Preparation according to preparation 1: 1-bromo-3-chloro-5-fluorobenzene (7.5 g, 35.9 mmol), tetrahydrofuran (100 ml), n-butyl lithium (2.5 M in hexane, 20 ml, 40 mmol), 4-boc-1-piperidone (7.8 g, 39.5 mmol). Yield: 8.75 g. MS m/z (rel. intensity, 70 eV) 329 (M+, 2), 255 (32), 211 (12), 157 (14), 57 (bp).

### Preparation 24:

### 4-(3-CHLORO-5-FLUOROPHENYL)-1,2,3,6-TETRAHYDROPYRIDINE

Preparation according to preparation 6: tert-butyl 4-(3-chloro-5-fluorophenyl)-4-hydroxy-piperidine-1-carboxylate (8.7 g, 26.4 mmol), trifluoroacetic acid (40 ml). Yield: 6.2 g. MS m/z (rel. intensity, 70 eV) 211 (M+, bp), 210 (39), 147 (77), 146 (55), 82 (44).

### Preparation 25:

### TERT-BUTYL 4-[4-CHLORO-3-(TRIFLUOROMETHYL)PHENYL]-4-HYDROXYPIPERIDINE-1-CARBOXYLATE

Preparation according to preparation 11: magnesium turnings (0.29 g, 7.7 mmol), 4-bromo-1-chloro-2-(trifluoromethyl)benzene (2.0 g, 7.7 mmol), 4-Boc-1-piperidone (1.53 g, 7.7 mmol), diethyl ether (40 ml). Yield: 1.6 g. MS m/z (rel. intensity, 70 eV) 379 (M+, 1), 261 (4), 207 (3), 57 (bp), 56 (21).

### Preparation 26:

### 4-[4-CHLORO-3-(TRIFLUOROMETHYL)PHENYL]-1,2,3,6-TETRAHYDROPYRIDINE

Preparation according to preparation 6: tert-butyl 4-[4-chloro-3-(trifluoromethyl)phenyl]-4-hydroxypiperidine-1-carboxylate (1.0 g, 2.64 mmol), trifluoroacetic acid (10 ml). Yield: 0.5 g. MS m/z (rel. intensity, 70 eV) 263(33), 262 (29), 261 (M+, bp), 260 (67), 257 (46).

### Preparation 27:

### 4-(2-CHLORO-4-FLUOROPHENYL)PYRIDINE

To a mixture of 1-bromo-2-chloro-4-fluorobenzene (2.0 g, 9.5 mmol), 1-pyridyl-4-boronic acid (1.3 g, 10.5 mmol), sodium carbonate (2.0 g, 23.9 mmol) and triphenylphosphine (0.5 g, 1.9 mmol) in toluene/ethanol (1:1, 60 ml) under nitrogen, was added tris(dibenzylideneacetone)dipalladium(0) (0.22 g , 2.5 mol%). The mixture was heated at reflux for 48 h, cooled to ambient temperature and diluted with water (50 ml) and ethyl acetate (100 ml). The organic layer was separated and the aqueous phase was extracted with ethyl acetate (2x50 ml). The combined organic phases was evaporated to dryness and dissolved in aqueous hydrochloric acid (10%, 50 ml). The solution was washed with diethyl ether (2x40 ml), basified with aqueous sodium hydroxide (2M) and extracted with ethyl acetate (2x50 ml). The combined organic phases was dried (MgSO4) and evaporated to dryness to give the title compound (1.16 g). MS m/z (rel. intensity, 70 eV) 209 (33), 207 (M+, bp), 208 (14), 172 (24), 145 (16).

The following tests were used for evaluation of the compounds according to the invention.

### In vivo test: Neurochemistry

Male Sprague-Dawley rats weighing 220-320g are used throughout the experiments. Sixty (60) minutes following administration of the test substance, the rats are decapitated. Directly after decapitation the brain is removed from the skull and put on a glass petri bowl filled with ice. The limbic system (containing the nucleus accumbens - both the core and shell, most parts of the olfactory tubercle and ventral pallidum) is dissected using two thin, angled tweezers and put directly on foil on dry ice (carbon dioxide -78° C). The striatum and cortex are then dissected and also put on dry ice. The time from decapitation until the last tissue is dissected varies from four to six minutes.. The tissue is weighed using a Sartorius BP3105 connected to a computer and packed in labelled tin foil, then stored in an -80° C freezer. Great care is taken in order to keep the tissue frozen until the time of neurochemical analysis. Each brain part is subsequently analyzed with respect to its content of monoamines and their metabolites.

The monoamine transmitter substances (NE (norepinephrine), DA (dopamine), 5-HT (serotonin)) as well as their amine (NM (normethanephrine), 3-MT (3-methoxytyramine)) and acid (DOPAC (3,4-dihydroxyphenylacetic acid), 5-HIAA (5-hydroxyindoleacetic acid), HVA (homovanillic acid)) metabolites are quantified in brain tissue homogenates by HPLC separations and electrochemical detection.

The analytical method is based on two chromatographic separations dedicated for amines or acids. Two chromatographic systems share a common auto injector with a 10-port valve and two sample loops for simultaneous injection on the two systems. Both systems are equipped with a reverse phase column (Luna C18(2), dp 3 µm, 50*2mm i.d., Phenomenex) and electrochemical detection is accomplished at two potentials on glassy carbon electrodes (MF-1000, Bioanalytical Systems, Inc.). The column effluent is passed *via* a T-connection to the detection cell or to a waste outlet. This is accomplished by two solenoid valves, which block either the waste or detector outlet. By preventing the chromatographic front from reaching the detector, better detection conditions are achieved. The aqueous mobile phase (0.4 ml/min) for the acid system contains citric acid 14 mM, sodium citrate 10 mM, MeOH 15% (v/v) and EDTA 0.1 mM. Detection potentials relative to Ag/AgCl reference are 0.45 and 0.60V. The aqueous ion pairing mobile phase (0.5 ml/min) for the amine system contains citric acid 5 mM, sodium citrate 10 mM, MeOH 9% (v/v), MeCN 10.5% (v/v), decane sulfonic acid 0.45 mM, and EDTA 0.1 mM. Detection potentials relative to Ag/AgCl reference are 0.45 and 0.65V.

### In vivo test: Microdialysis

Male Sprague-Dawley rats weighing 220-320g were used throughout the experiments. Before the experiment the animals were group housed, five animals in each cage, with free access to water and food. The animals were housed at least one week after arrival prior to surgery and use in the experiments. Each rat was used only once for microdialysis.

We use a modified version (Waters, Lofberg et al. 1994) of the I-shaped probe (Santiago and Westerink 1990). The dialysis membrane we use is the AN69 polyacrylonitrile/ sodium methalylsulfonate copolymer (HOSPAL; o.d./i.d. 310/220 µm: Gambro, Lund, Sweden). In the dorsal striatum we use probes with an exposed length of 3 mm of dialysis membrane and in the prefrontal cortex the corresponding length is 2.5 mm. The rats were operated under isoflurane inhalation anesthesia while mounted into a Kopf stereotaxic instrument. Coordinates were calculated relative to bregma; dorsal striatum AP +1, ML ± 2.6, DV - 6.3; Pf cortex, AP +3,2 , 8° ML ±1,2 , DV - 4,0 .according to (Paxinos and Watson 1986). The dialysis probe was positioned in a burr hole under stereotaxic guidance and cemented with phosphatine dental cement.

The rats were housed individually in cages for 48 h before the dialysis experiments, allowing them to recover from surgery and minimizing the risk of drug interactions with the anaesthetic during the following experiments. During this period the rats had free access to food and water. On the day of experiment the rats were connected to a micro perfusion pump via a swivel and were replaced in the cage where they could move freely within its confinements. The perfusion medium was a Ringer's solution containing in mmol/l: NaCl; 140, CaCl2; 1.2, KCl; 3.0, MgCl2; 1.0 and ascorbic acid; 0.04 according to (Moghaddam and Bunney 1989). The pump was set to a perfusion speed of 2 µl/min and 40 µl samples were collected every 20 min.

30 µl of each sample was injected into the chromatograph. On a 10-port injector (Valco C10W), with two sample loops mounted in series (2µl and 20µl), each brain dialysate sample is loaded in both loops simultaneously. When the valve is switched to inject the main part of the 20 µl sample is introduced into a reverse-phase ion-pairing system for dopamine (DA), norepinephrine (NE), normetanephrine (NM), 3-methoxytyramine(3-MT) and serotonin (5-hydroxytryptamine, 5-HT) determination (large loop), while a small fraction (2 µl, from the small loop) is introduced on a reverse-phase column for the chromatography of the acidic monoamine metabolites 3,4-di-hydroxyphenylacetic acid (DOPAC), homovanillic acid (HVA) and 5-hydroxyindoleacetic acid (5-HIAA). The currents generated by the two EC detectors are converted to digital data and evaluated using Chromelion software (Dionex, Sunnyvale, California) on a PC. The method sample turn over time was 4.5 min and two parallel experiments are normally analysed simultaneously on the system. After the experiment the rats were uncoupled from the perfusion pump and decapitated. Their brains were rapidly taken out and fixed in Neo-fix solution (Kebolab, Sweden) for subsequent inspection of probe localisation. The Animal Ethics Committee in Göteborg, Sweden approved the procedures applied in these experiments.

Moghaddam, B. and B. S. Bunney (1989). "Ionic Composition of Microdialysis Perfusing Solution Alters the Pharmacological Responsiveness and Basal Outflow of Striatal Dopamine." J. Neurochem. 53: 652-654.

Paxinos, G. and C. Watson (1986). The Rat Brain in Stereotaxic Coordinates. New York, Academic Press.

Santiago, M. and B. H. C. Westerink (1990). "Characterization of the in vivo release of dopamine as recorded by different types of intracerebral microdialysis probes." Naunyn-Schmiedeberg's Arch. Pharmacol. 342: 407-414.

Waters, N., L. Lofberg, S. Haadsma-Svensson, K. Svensson, C. Sonesson and A. Carlsson (1994). "Differential effects of dopamine D2 and D3 receptor antagonists in regard to dopamine release, in vivo receptor displacement and behaviour." J Neural Transm Gen Sect 98(1): 39-55.

## Claims

1. A compound of formula (4): 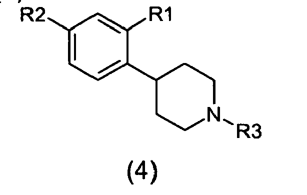 wherein:
R₁ is selected from the group consisting of -CN, -CF₃, -CHF₂, F and Cl;
R₂ is selected from the group consisting of F and Cl;
R₃ is H
and the pharmaceutically acceptable salts thereof,
or a compound of formula (5): 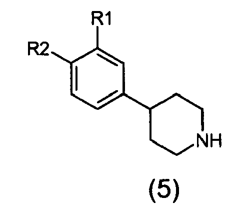 wherein:
R₁ is selected from the group consisting of -CN, -CF₃, -CHF₂, F and Cl;
R₂ is selected from the group consisting of F and Cl;
and the pharmaceutically acceptable salts thereof,
or a compound of formula (6): 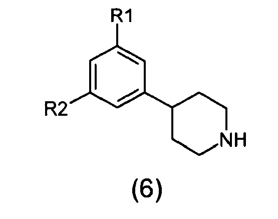 wherein:
R₁ is selected from the group consisting of -F and Cl;
R₂ is selected from the group consisting of F;
and the pharmaceutically acceptable salts thereof,
with the provisos that;
in Formula (4) above,
R₁ and R₂ are not both F when R₃ is H;
R₁ and R₂ are not both Cl when R₃ is H;
R₁ is not -CN when R₂ is F and R₃ is H;
R₁ is not -CN when R₂ is Cl and R₃ is H;
R₁ is not Cl when R₂ is F and R₃ is H;
in Formula (5) above,
R₁ and R₂ are not both F;
R₁ and R₂ are not both Cl;
R₁ is not -CF₃ when R₂ is Cl;
R₁ is not F when R₂ is Cl ;
R₁ is not -CF₃ when R₂ is F;
and in Formula (6) above,
R₁ and R₂ are not both F;

2. A compound according to claim 1, wherein R₁ is F or Cl.

3. A compound according to any of claims 1-2, wherein R₁ is F.

4. A compound according to any of claims 1-3, wherein R₂ is F or Cl.

5. A compound according to any of claims 1-4, wherein R₂ is F.

6. A compound according to claim 1, selected from the group consisting of:
4-(3-CHLORO-5-FLUOROPHENYL)PIPERIDINE
4-(3-CHLORO-4-FLUOROPHENYL)PIPERIDINE
4-(4-CHLORO-2-FLUOROPHENYL)PIPERIDINE
2-FLUORO-5-PIPERIDIN-4-YL-BENZONITRILE
2-CHLORO-5-PIPERIDIN-4-YL-BENZONITRILE
4-[3-(DIFLUOROMETHYL)-4-FLUOROPHENYL]PIPERIDINE
4-[4-CHLORO-3-(DIFLUOROMETHYL)PHENYL]PIPERIDINE
4-[2-(DIFLUOROMETHYL)-4-FLUOROPHENYL]PIPERIDINE
4-[4-CHLORO-2-(DIFLUOROMETHYL)PHENYL]PIPERIDINE
4-[4-FLUORO-2-(TRIFLUOROMETHYL)PHENYL]-PIPERIDINE
4-[4-CHLORO-2-(TRIFLUOROMETHYL)PHENYL]-PIPERIDINE

7. A compound according to any of claims 1-6 for use as a medicament.

8. Use of a compound of formula (4): 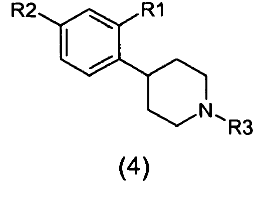 wherein:
R₁ is selected from the group consisting of -CN, -CF₃, -CHF₂, F and Cl;
R₂ is selected from the group consisting of F and Cl;
R₃ is H
and the pharmaceutically acceptable salts thereof,
or a compound of formula (5): 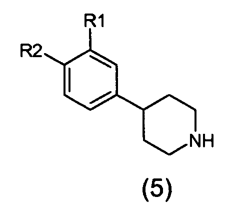 wherein:
R₁ is selected from the group consisting of -CN, -CF₃, -CHF₂, F and Cl;
R₂ is selected from the group consisting of F and Cl;
and the pharmaceutically acceptable salts thereof,
or a compound of formula (6): 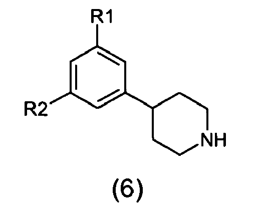 wherein:
R₁ is selected from the group consisting of -F and Cl;
R₂ is selected from the group consisting of F;
and the pharmaceutically acceptable salts thereof, in the manufacture of a medicament for the treatment of a disorder of the central nervous system.

9. Use according to claim 8, wherein R₁ is F or Cl.

10. Use according to any of claims 8-9, wherein R₁ is F.

11. Use according to any of claims 8-10, wherein R₂ is F or Cl.

12. Use according to any of claims 8-11, wherein R₂ is F.

13. Use according to claim 8, wherein the compound is selected from the group consisting of:
4-(3,5-DIFLUOROPHENYL)PIPERIDINE
4-(3,4-DIFLUOROPHENYL)PIPERIDINE
4-(3,4-DICHLOROPHENYL)PIPERIDINE
4-(4-CHLORO-3-FLUOROPHENYL)PIPERIDINE
4-(2,4-DIFLUOROPHENYL)PIPERIDINE
4-(2,4-DICHLOROPHENYL)PIPERIDINE
4-(2-CHLORO-4-FLUOROPHENYL)PIPERIDINE
5-FLUORO-2-PIPERIDIN-4-YL-BENZONITRILE
5-CHLORO-2-PIPERIDIN-4-YL-BENZONITRILE
4-[4-CHLORO-3-(TRIFLUOROMETHYL)PHENYL]-PIPERIDINE
4-[4-FLUORO-3-(TRIFLUOROMETHYL)PHENYL]-PIPERIDINE

14. Use of a compound according to any of claims 1-7 and the pharmaceutically acceptable salts thereof, in the manufacture of a medicament for the treatment of a disorder of the central nervous system.

15. Use according to any of claims 8-14, wherein the disorder of the central nervous system is a psychiatric disorder.

16. Use according to any of claims 8-14, wherein the disorder of the central nervous system is a cognitive disorder, such as e.g. a neurodegenerative disorder (e.g. dementia and age-related cognitive impairment) or developmental disorder (e.g. Autism spectrum disorders, ADHD, Cerebral Palsy, Gilles de la Tourette's syndrome) or a cognitive disorders occurring as part of the core symptoms of schizophrenia.

17. Use according to any of claims 8-14, wherein the disorder of the central nervous system is schizophrenia and schizophreniform disorders.

18. Use according to any of claims 8-14, wherein the disorder of the central nervous system is an affective disorder, such as depression or bipolar disorder.

19. Use according to any of claims 8-14, wherein the disorder of the central nervous system is an anxiety disorder, such as generalized anxiety disorder (GAD), specific phobias or panic disorder (PD).

20. Use according to any of claims 8-14, wherein the disorder of the central nervous system is a sleep disorder.

21. A pharmaceutical composition comprising a compound according to any of claims 1-7 and one or more pharmaceutically acceptable carriers or diluents.

22. A pharmaceutical composition according to claim 21, for the treatment of a disorder of the central nervous system.

23. A pharmaceutical composition according to any of claims 21-22, for the treatment of a disorder defined in any one of claims 14-20.

24. A compound of formula (4): 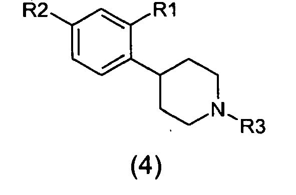 wherein:
R₁ is selected from the group consisting of -CN, -CF₃, -CHF₂, F and Cl;
R₂ is selected from the group consisting of F and Cl;
R₃ is H
and the pharmaceutically acceptable salts thereof,
or a compound of formula (5): 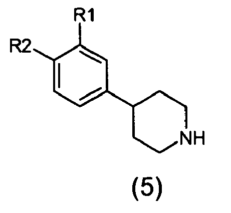 wherein:
R₁ is selected from the group consisting of -CN, -CF₃, -CHF₂, F and Cl;
R₂ is selected from the group consisting of F and Cl;
and the pharmaceutically acceptable salts thereof,
or a compound of formula (6): 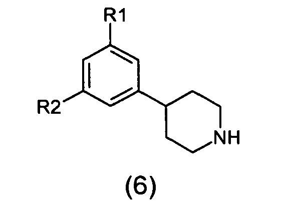 wherein:
R₁ is selected from the group consisting of -F and Cl;
R₂ is selected from the group consisting of F;
and the pharmaceutically acceptable salts thereof,
for use as a medicament.

25. A compound of formula (4): 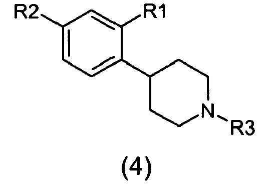 wherein:
R₁ is selected from the group consisting of -CN, -CF₃, -CHF₂, F and Cl;
R₂ is selected from the group consisting of F and Cl;
R₃ is H
and the pharmaceutically acceptable salts thereof,
or a compound of formula (5): 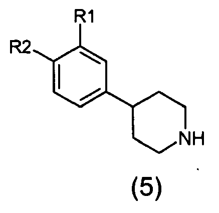 wherein:
R₁ is selected from the group consisting of -CN, -CF₃, -CHF₂, F and Cl;
R₂ is selected from the group consisting of F and Cl;
and the pharmaceutically acceptable salts thereof,
or a compound of formula (6): 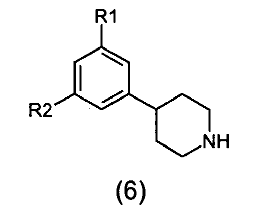 wherein:
R₁ is selected from the group consisting of -F and Cl;
R₂ is selected from the group consisting of F;
and the pharmaceutically acceptable salts thereof,
for use in treatment of a disorder of the central nervous system.

## Patentansprüche

1. Verbindung der Formel (4): 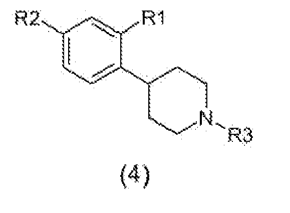 wobei:
R₁ ausgewählt ist aus der Gruppe bestehend aus -CN, -CF₃, -CHF₂, F und Cl;
R₂ ausgewählt ist aus der Gruppe bestehend aus F und Cl;
R₃ H ist
und die pharmazeutisch verträglichen Salze davon,
oder eine Verbindung der Formel (5): 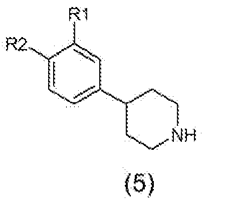 wobei:
R₁ ausgewählt ist aus der Gruppe bestehend aus -CN, -CF₃, -CHF₂, F und Cl;
R₂ ausgewählt ist aus der Gruppe bestehend aus F und Cl;
und die pharmazeutisch verträglichen Salze davon,
oder eine Verbindung der Formel (6): 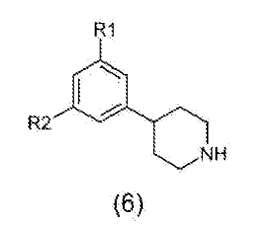 wobei:
R₁ ausgewählt ist aus der Gruppe bestehend aus -F und Cl;
R₂ ausgewählt ist aus der Gruppe bestehend aus F;
und die pharmazeutisch verträglichen Salze davon,
mit den Maßgaben, dass
in vorstehender Formel (4)
R₁ und R₂ nicht beide F sind, wenn R₃ H ist;
R₁ und R₂ nicht beide Cl ist, wenn R₃ H ist;
R₁ nicht -CN ist, wenn R₂ F ist und R₃ H ist;
R₁ nicht -CN ist, wenn R₂ Cl ist und R₃ H ist;
R₁ nicht Cl ist, wenn R₂ F ist und R₃ H ist;
in vorstehender Formel (5)
R₁ und R₂ nicht beide F sind;
R₁ und R₂ nicht beide Cl sind;
R₁ nicht -CF₃ ist, wenn R₂ Cl ist;
R₁ nicht F ist, wenn R₂ Cl ist;
R₁ nicht -CF₃ ist, wenn R₂ F ist;
und in vorstehender Formel (6)
R₁ und R₂ nicht beide F sind.

2. Verbindung nach Anspruch 1, wobei R₁ F oder Cl ist.

3. Verbindung nach einem der Ansprüche 1-2, wobei R₁ F ist.

4. Verbindung nach einem der Ansprüche 1-3, wobei R₂ F oder Cl ist.

5. Verbindung nach einem der Ansprüche 1-4, wobei R₂ F ist.

6. Verbindung nach Anspruch 1, ausgewählt aus der Gruppe bestehend aus:
4-(3-CHLOR-5-FLUORPHENYL)PIPERIDIN
4-(3-CHLOR-4-FLUORPHENYL)PIPERIDIN
4-(4-CHLOR-2-FLUORPHENYL)PIPERIDIN
2-FLUOR-5-PIPERIDIN-4-YL-BENZONITRIL 2-CHLOR-5-PIPERIDIN-4-YL-BENZONITRIL
4-[3-(DIFLUORMETHYL)-4-FLUORPHENYL]PIPERIDIN
4-[4-CHLOR-3-(DIFLUORMETHYL)PHENYL]PIPERIDIN
4-[2-(DIFLUORMETHYL)-4-FLUORPHENYL]PIPERIDIN
4-[4-CHLOR-2-(DIFLUORMETHYL)PHENYL]PIPERIDIN
4-[4-FLUOR-2-(TRIFLUORMETHYL)PHENYL]-PIPERIDIN
4-[4-CHLOR-2-(TRIFLUORMETHYL)PHENYL]-PIPERIDIN

7. Verbindung nach einem der Ansprüche 1-6 zur Verwendung als ein Medikament.

8. Verwendung einer Verbindung der Formel (4) 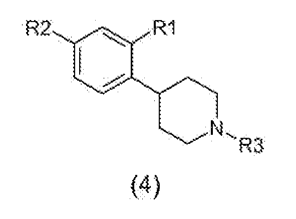 wobei:
R₁ ausgewählt ist aus der Gruppe bestehend aus -CN, -CF₃, -CHF₂, F und Cl;
R₂ ausgewählt ist aus der Gruppe bestehend aus F und Cl;
R₃ H ist
und der pharmazeutisch verträglichen Salze davon,
oder einer Verbindung der Formel (5): 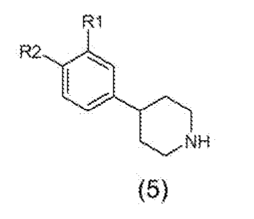 wobei:
R₁ ausgewählt ist aus der Gruppe bestehend aus -CN, -CF₃, -CHF₂, F und Cl;
R₂ ausgewählt ist aus der Gruppe bestehend aus F und Cl;
und der pharmazeutisch verträglichen Salze davon,
oder einer Verbindung der Formel (6): 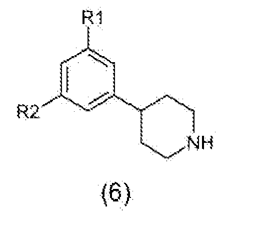 wobei:
R₁ ausgewählt ist aus der Gruppe bestehend aus -F und Cl;
R₂ ausgewählt ist aus der Gruppe bestehend aus F;
und der pharmazeutisch verträglichen Salze davon bei der Herstellung eines Medikaments für die Behandlung einer Störung des Zentralnervensystems.

9. Verwendung nach Anspruch 8, wobei R₁ F oder Cl ist.

10. Verwendung nach einem der Ansprüche 8-9, wobei R₁ F ist.

11. Verwendung nach einem der Ansprüche 8-10, wobei R₂ F oder Cl ist.

12. Verwendung nach einem der Ansprüche 8-11, wobei R₂ F ist.

13. Verwendung nach Anspruch 8, wobei die Verbindung ausgewählt ist aus der Gruppe bestehend aus:
4-(3,5-DIFLUORPHENYL)PIPERIDIN
4-(3,4-DIFLUORPHENYL)PIPERIDIN
4-(3,4-DICHLORPHENYL)PIPERIDIN
4-(4-CHLOR-3-FLUORPHENYL)PIPERIDIN
4-(2,4-DIFLUORPHENYL)PIPERIDIN
4-(2,4-DICHLORPHENYL)PIPERIDIN
4-(2-CHLOR-4-FLUORPHENYL)PIPERIDIN
5-FLUOR-2-PIPERIDIN-4-YL-BENZONITRIL
5-CHLOR-2-PIPERIDIN-4-YL-BENZONITRIL
4-[4-CHLOR-3-(TRIFLUORMETHYL)PHENYL]-PIPERIDIN
4-[4-FLUOR-3-(TRIFLUORMETHYL)PHENYL]-PIPERIDIN

14. Verwendung einer Verbindung nach einem der Ansprüche 1-7 und der pharmazeutisch verträglichen Salze davon bei der Herstellung eines Medikaments für die Behandlung einer Störung des Zentralnervensystems.

15. Verwendung nach einem der Ansprüche 8-14, wobei es sich bei der Störung des Zentralnervensystems um eine psychiatrische Störung handelt.

16. Verwendung nach einem der Ansprüche 8-14, wobei es sich bei der Störung des Zentralnervensystems um eine kognitive Störung, wie z.B. eine neurodegenerative Störung (z.B. Demenz und altersbedingte kognitive Beeinträchtigung) oder eine Entwicklungsstörung (z.B. Autismus-Spektrum-Störungen, ADHD, Zerebralparese, Gilles de la Tourette-Syndrom) oder eine kognitive Störung, die als Teil der Kernsymptome von Schizophrenie auftritt, handelt.

17. Verwendung nach einem der Ansprüche 8-14, wobei es sich bei der Störung des Zentralnervensystems um Schizophrenie und schizophreniforme Störungen handelt.

18. Verwendung nach einem der Ansprüche 8-14, wobei es sich bei der Störung des Zentralnervensystems um eine affektive Störung wie etwa eine Depression oder eine bipolare Störung handelt.

19. Verwendung nach einem der Ansprüche 8-14, wobei es sich bei der Störung des Zentralnervensystems um eine Angststörung, wie etwa eine generalisierte Angststörung (GAD), spezifische Phobien oder eine Panikstörung (PD) handelt.

20. Verwendung nach einem der Ansprüche 8-14, wobei es sich bei der Störung des Zentralnervensystems um eine Schlafstörung handelt.

21. Pharmazeutische Zusammensetzung umfassend eine Verbindung nach einem der Ansprüche 1-7 und einen oder mehrere pharmazeutisch verträgliche Träger oder Verdünnungsmittel.

22. Pharmazeutische Zusammensetzung nach Anspruch 21 für die Behandlung einer Störung des Zentralnervensystems.

23. Pharmazeutische Zusammensetzung nach einem der Ansprüche 21-22 für die Behandlung einer Störung, die in einem der Ansprüche 14-20 definiert ist.

24. Verbindung der Formel (4): 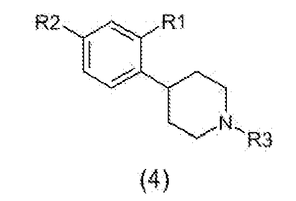 wobei:
R₁ ausgewählt ist aus der Gruppe bestehend aus -CN, -CF₃, -CHF₂, F und Cl;
R₂ ausgewählt ist aus der Gruppe bestehend aus F und Cl;
R₃ H ist
und die pharmazeutisch verträglichen Salze davon,
oder eine Verbindung der Formel (5): 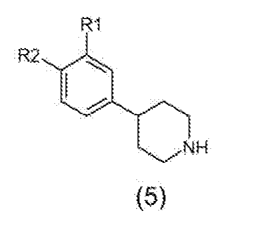 wobei:
R₁ ausgewählt ist aus der Gruppe bestehend aus -CN, -CF₃, -CHF₂, F und Cl;
R₂ ausgewählt ist aus der Gruppe bestehend aus F und Cl;
und die pharmazeutisch verträglichen Salze davon,
oder eine Verbindung der Formel (6): 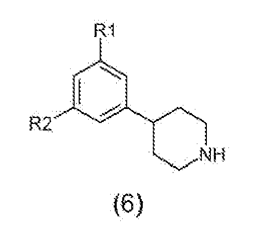 wobei:
R₁ ausgewählt ist aus der Gruppe bestehend aus -F und Cl;
R₂ ausgewählt ist aus der Gruppe bestehend aus F;
und die pharmazeutisch verträglichen Salze davon,
zur Verwendung als ein Medikament.

25. Verbindung der Formel (4): 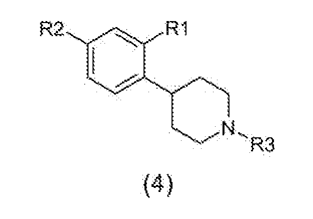 wobei:
R₁ ausgewählt ist aus der Gruppe bestehend aus -CN, -CF₃, -CHF₂, F und Cl;
R₂ ausgewählt ist aus der Gruppe bestehend aus F und Cl;
R₃ H ist
und die pharmazeutisch verträglichen Salze davon,
oder eine Verbindung der Formel (5): 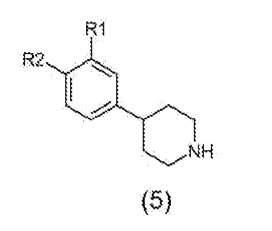 wobei:
R₁ ausgewählt ist aus der Gruppe bestehend aus -CN, -CF₃, -CHF₂, F und Cl;
R₂ ausgewählt ist aus der Gruppe bestehend aus F und Cl;
und die pharmazeutisch verträglichen Salze davon,
oder eine Verbindung der Formel (6): 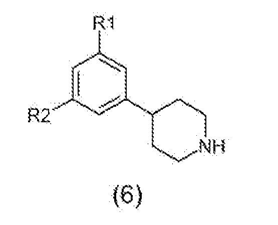 wobei:
R₁ ausgewählt ist aus der Gruppe bestehend aus -F und Cl;
R₂ ausgewählt ist aus der Gruppe bestehend aus F;
und die pharmazeutisch verträglichen Salze davon,
zur Verwendung bei der Behandlung einer Störung des Zentralnervensystems.

## Revendications

1. Composé de formule (4) : 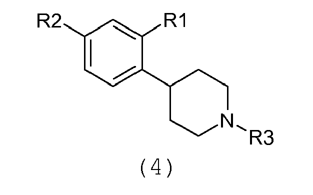 dans laquelle :
R₁ est choisi dans le groupe constitué par -CN, -CF₃, -CHF₂, F et Cl ;
R₂ est choisi dans le groupe constitué par F et Cl ;
R₃ est H
et les sels pharmaceutiquement acceptables de celui-ci,
ou composé de formule (5) : 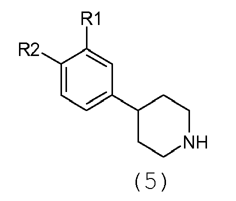 dans laquelle :
R₁ est choisi dans le groupe constitué par -CN, -CF₃, -CHF₂, F et Cl ;
R₂ est choisi dans le groupe constitué par F et Cl ;
et les sels pharmaceutiquement acceptables de celui-ci,
ou composé de formule (6) : 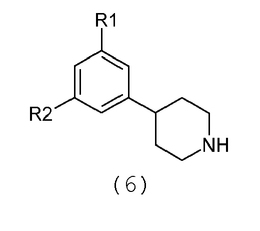 dans laquelle :
R₁ est choisi dans le groupe constitué par -F et Cl ;
R₂ est choisi dans le groupe constitué par F ;
et les sels pharmaceutiquement acceptables de celui-ci, avec pour conditions que ;
dans la formule (4) ci-dessus,
R₁ et R₂ ne sont pas tous les deux F lorsque R₃ est H ;
R₁ et R₂ ne sont pas tous les deux Cl lorsque R₃ est H ;
R₁ n'est pas -CN lorsque R₂ est F et R₃ est H ;
R₁ n'est pas -CN lorsque R₂ est Cl et R₃ est H ;
R₁ n'est pas Cl lorsque R₂ est F et R₃ est H ;
dans la formule (5) ci-dessus,
R₁ et R₂ ne sont pas tous les deux F ;
R₁ et R₂ ne sont pas tous les deux Cl ;
R₁ n'est pas -CF₃ lorsque R₂ est Cl ;
R₁ n'est pas F lorsque R₂ est Cl ;
R₁ n'est pas -CF₃ lorsque R₂ est F ;
et dans la formule (6) ci-dessus,
R₁ et R₂ ne sont pas tous les deux F.

2. Composé selon la revendication 1, dans lequel R₁ est F ou Cl.

3. Composé selon l'une quelconque des revendications 1-2, dans lequel R₁ est F.

4. Composé selon l'une quelconque des revendications 1-3, dans lequel R₂ est F ou Cl.

5. Composé selon l'une quelconque des revendications 1-4, dans lequel R₂ est F.

6. Composé selon la revendication 1, choisi dans le groupe constitué par les :
4-(3-CHLORO-5-FLUOROPHENYL)PIPERIDINE
4-(3-CHLORO-4-FLUOROPHENYL)PIPERIDINE
4-(4-CHLORO-2-FLUOROPHENYL)PIPERIDINE
2-FLUORO-5-PIPERIDIN-4-YL-BENZONITRILE
2-CHLORO-5-PIPERIDIN-4-YL-BENZONITRILE
4-[3-(DIFLUOROMETHYL)-4-FLUOROPHENYL]PIPERIDINE
4-[4-CHLORO-3-(DIFLUOROMETHYL)PHENYL]PIPERIDINE
4-[2-(DIFLUOROMETHYL)-4-FLUOROPHENYL]PIPERIDINE
4-[4-CHLORO-2-(DIFLUOROMETHYL)PHENYL]PIPERIDINE
4-[4-FLUORO-2-(TRIFLUOROMETHYL)PHENYL]-PIPERIDINE
4-[4-CHLORO-2-(TRIFLUOROMETHYL)PHENYL]-PIPERIDINE

7. Composé selon l'une quelconque des revendications 1-6, pour l'utilisation en tant que médicament.

8. Utilisation d'un composé de formule (4) : 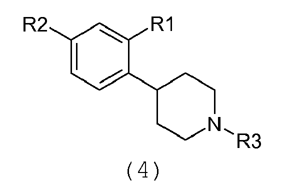 dans laquelle :
R₁ est choisi dans le groupe constitué par -CN, -CF₃, -CHF₂, F et Cl ;
R₂ est choisi dans le groupe constitué par F et Cl ;
R₃ est H
et des sels pharmaceutiquement acceptables de celui-ci,
ou d'un composé de formule (5) : 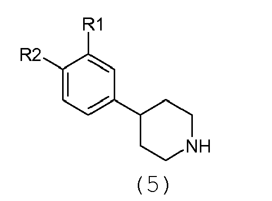 dans laquelle :
R₁ est choisi dans le groupe constitué par -CN, -CF₃, -CHF₂, F et Cl ;
R₂ est choisi dans le groupe constitué par F et Cl ;
et des sels pharmaceutiquement acceptables de celui-ci,
ou d'un composé de formule (6) : 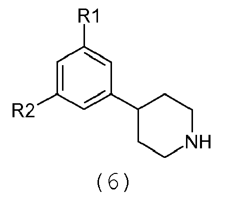 dans laquelle :
R₁ est choisi dans le groupe constitué par -F et Cl ;
R₂ est choisi dans le groupe constitué par F ;
et des sels pharmaceutiquement acceptables de celui-ci, dans la fabrication d'un médicament pour le traitement d'un trouble du système nerveux central.

9. Utilisation selon la revendication 8, dans laquelle R₁ est F ou Cl.

10. Utilisation selon l'une quelconque des revendications 8-9, dans laquelle R₁ est F.

11. Utilisation selon l'une quelconque des revendications 8-10, dans laquelle R₂ est F ou Cl.

12. Utilisation selon l'une quelconque des revendications 8-11, dans laquelle R₂ est F.

13. Utilisation selon la revendication 8, dans laquelle le composé est choisi dans le groupe constitué par les :
4-(3,5-DIFLUOROPHENYL)PIPERIDINE
4-(3,4-DIFLUOROPHENYL)PIPERIDINE
4-(3,4-DICHLOROPHENYL)PIPERIDINE
4-(4-CHLORO-3-FLUOROPHENYL)PIPERIDINE
4-(2,4-DIFLUOROPHENYL)PIPERIDINE
4-(2,4-DICHLOROPHENYL)PIPERIDINE
4-(2-CHLORO-4-FLUOROPHENYL)PIPERIDINE
5-FLUORO-2-PIPERIDIN-4-YL-BENZONITRILE
5-CHLORO-2-PIPERIDIN-4-YL-BENZONITRILE
4-[4-CHLORO-3-(TRIFLUOROMETHYL)PHENYL]-PIPERIDINE
4-[4-FLUORO-3-(TRIFLUOROMETHYL)PHENYL]-PIPERIDINE

14. Utilisation d'un composé selon l'une quelconque des revendications 1-7 et des sels pharmaceutiquement acceptables de celui-ci, dans la fabrication d'un médicament pour le traitement d'un trouble du système nerveux central.

15. Utilisation selon l'une quelconque des revendications 8-14, dans laquelle le trouble du système nerveux central est un trouble psychiatrique.

16. Utilisation selon l'une quelconque des revendications 8-14, dans laquelle le trouble du système nerveux central est un trouble cognitif tel que, par exemple, un trouble neurodégénératif (par exemple, une démence et une détérioration cognitive liée à l'âge) ou un trouble développemental (par exemple, des troubles du spectre de l'autisme, un THADA, une infirmité motrice cérébrale, un syndrome de Gilles de la Tourette) ou un trouble cognitif se produisant en tant que partie des symptômes au coeur de la schizophrénie.

17. Utilisation selon l'une quelconque des revendications 8-14, dans laquelle le trouble du système nerveux central est une schizophrénie et des troubles schizophréniformes.

18. Utilisation selon l'une quelconque des revendications 8-14, dans laquelle le trouble du système nerveux central est un trouble affectif, tel qu'une dépression ou un trouble bipolaire.

19. Utilisation selon l'une quelconque des revendications 8-14, dans laquelle le trouble du système nerveux central est un trouble d'anxiété, tel qu'un trouble d'anxiété généralisée (GAD), des phobies spécifiques ou un trouble panique (PD).

20. Utilisation selon l'une quelconque des revendications 8-14, dans laquelle le trouble du système nerveux central est un trouble du sommeil.

21. Composition pharmaceutique comprenant un composé selon l'une quelconque des revendications 1-7 et un ou plusieurs véhicules ou diluants pharmaceutiquement acceptables.

22. Composition pharmaceutique selon la revendication 21, pour le traitement d'un trouble du système nerveux central.

23. Composition pharmaceutique selon l'une quelconque des revendications 21-22, pour le traitement d'un trouble défini dans l'une quelconque des revendications 14-20.

24. Composé de formule (4) : 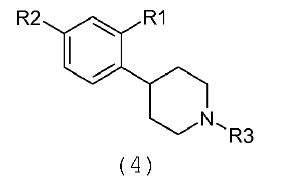 dans laquelle :
R₁ est choisi dans le groupe constitué par -CN, -CF₃, -CHF₂, F et Cl ;
R₂ est choisi dans le groupe constitué par F et Cl ;
R₃ est H
et les sels pharmaceutiquement acceptables de celui-ci,
ou composé de formule (5) : 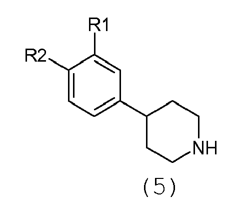 dans laquelle :
R₁ est choisi dans le groupe constitué par -CN, -CF₃, -CHF₂, F et Cl ;
R₂ est choisi dans le groupe constitué par F et Cl ;
et les sels pharmaceutiquement acceptables de celui-ci,
ou composé de formule (6) : 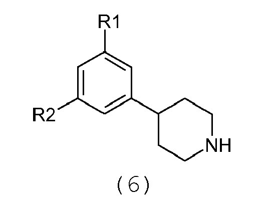 dans laquelle :
R₁ est choisi dans le groupe constitué par -F et Cl ;
R₂ est choisi dans le groupe constitué par F ;
et les sels pharmaceutiquement acceptables de celui-ci, pour l'utilisation en tant que médicament.

25. Composé de formule (4) : 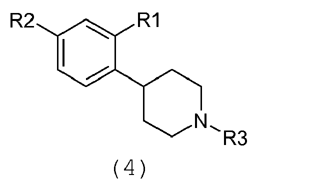 dans laquelle :
R₁ est choisi dans le groupe constitué par -CN, -CF₃, -CHF₂, F et Cl ;
R₂ est choisi dans le groupe constitué par F et Cl ;
R₃ est H
et les sels pharmaceutiquement acceptables de celui-ci,
ou composé de formule (5) : 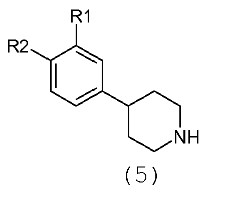 dans laquelle :
R₁ est choisi dans le groupe constitué par -CN, -CF₃, -CHF₂, F et Cl ;
R₂ est choisi dans le groupe constitué par F et Cl ;
et les sels pharmaceutiquement acceptables de celui-ci,
ou composé de formule (6) : 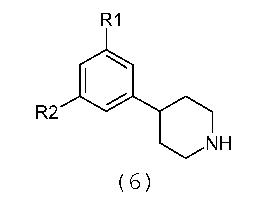 dans laquelle :
R₁ est choisi dans le groupe constitué par -F et Cl ;
R₂ est choisi dans le groupe constitué par F ;
et les sels pharmaceutiquement acceptables de celui-ci, pour l'utilisation dans le traitement d'un trouble du système nerveux central.
